# EUROPEAN PATENT APPLICATION

(11) **EP 2 930 234 A1**
(43) Date of publication of application: **14.10.2015**
(21) Application number: 13860224.8
(22) Date of filing: 03.12.2013
(51) Int. Cl.: C12N 1/12, A01G 33/00

(54) **METHOD FOR COLLECTING SEED ALGAE FROM MICROALGAE ON LIQUID SURFACE, AND FOR PERFORMING CULTURING IN SEPARATE CULTURE VESSEL, IN METHOD FOR CULTURING MICROALGAE ON LIQUID SURFACE**

(30) Priority: 07.12.2012 JP 2012268781
(71) Applicant: FUJIFILM Corporation, Tokyo 106-8620 (JP)
(72) Inventor: KANEHARA, Hideyuki, Ashigarakami-gun Kanagawa 258-8577 (JP); MATSUNAGA, Tadashi, Fuchu-shi Tokyo 183-8538 (JP); TANAKA, Tsuyoshi, Fuchu-shi Tokyo 183-8538 (JP); TANAKA, Masayoshi, Meguro-ku Tokyo (JP)
(74) Representative: Hoffmann Eitle
(86) International application number: PCT/JP2013/082500
(87) International publication number: WO 2014/088010

(57) **Abstract**

Provided is a method of culturing microalgae which can reduce the production cost of biomass derived from microalgae.

There is provided a method of culturing microalgae, including: culturing in a first stage in which microalgae obtained through a purification process are cultured in a culture solution within a first culture container to form a biofilm as a film-like structure or a three-dimensional structure which is formed of the microalgae on a liquid surface of the culture solution; and
culturing in a second stage in which microalgae are cultured on a liquid surface using at least a part of the biofilm as a film-like structure or a three-dimensional structure which is formed on the liquid surface of the culture solution, as seed algae.

## Description

### Technical Field

The present invention relates to a method of collecting seed algae from microalgae on a liquid surface and of performing culturing in a separate culture container, in a method of culturing microalgae on a liquid surface. More specifically, the present invention relates to the method of culturing microalgae; a biofilm formed on the liquid surface through the culturing method; biomass and oil obtained from the biofilm; and a method of producing biomass derived from microalgae. The method of culturing microalgae according to the present invention is useful in energy-related fields since a biofilm is formed on a liquid surface.

### Background Art

In recent years, there has been a problem in that an increase in fuel prices due to use of a large amount of fossil fuels, or global warming due to the greenhouse effect caused by carbon dioxide released into the air due to the use of fossil fuel being in progress, or exhaustion of the fossil fuel, accompanying development of industrial activities. As means for solving these problems, there are growing expectations for utilization of microalgae having an ability of fixing carbon dioxide using light energy to convert it into hydrocarbon compounds, biodiesel (triglycerides), or the like. Various research, in which carbon dioxide is fixed using light energy by, for example, culturing microalgae, which produces hydrocarbon compounds, to produce biomass such as biodiesel or hydrocarbon compounds, have already been conducted.

As an existence form of microorganisms such as microalgae, for example, there is an existence form of producing viscous secretions to form a highly ordered structure, that is, a biofilm (microorganism aggregate) for protecting themselves from external attack, and such an existence form has recently been attracting great attention in the medical or environmental fields or the like. It is known that in a biofilm, when formed, microorganisms show behavior which is different from individual properties thereof, that is, show properties as an aggregate. For example, the biofilm forms a physical barrier, which makes preying of a predatory organism on the microorganisms difficult compared to when the microorganisms independently exist and makes substitution of the microorganisms by other microorganisms difficult.

There are various problems in producing biomass using microalgae. Moreover, efficient methods of culturing and collecting microalgae and extracting biomass such as oil have not been developed, and the cost of producing biomass is high. Therefore, the production thereof has not been carried out on a commercial scale. One of the biggest reasons thereof is that there is no efficient method of collecting the microalgae. Specifically, microalgae are generally grown while floating in liquid. Therefore, in order to use the microalgae as biomass, microalgae need to be collected from a large amount of liquid. In addition, light energy is required for growth of microalgae. Therefore, it is impossible to excessively increase the concentration of the microalgae existing in the liquid in order to secure sufficient irradiation with light. As a result, it is necessary to filter a large amount of water in order to collect the microalgae floating in the liquid. In addition, the microalgae are generally small in size, and thus are difficult to be filtered. Although a method of using a precipitant, a method of using a centrifugal separator, and a method of using microalgae as bait for larger organisms and collecting the larger organisms have been attempted in order to study collecting methods for solving such problems, no methods have led to a fundamental solution.

Due to the above-described reasons, it is desirable that microalgae are grown on a liquid surface in order to efficiently and simply collect the microalgae at a low cost.

Examples of growing microalgae on a liquid surface include blooming (massive proliferation) of Microcystis and Botryococcus that occurs naturally, and red tide that occurs in the ocean. However, all of these are phenomena that occur in nature, and various kinds and a large number of impurities are mixed with microalgae generated on a liquid surface. Therefore, it is not certain whether pure microalgae grow on the liquid surface. Microcystis is referred to as an aggregate which is formed of microalgae that are not subjected to purification, that is, formed of various kinds of microalgae; which floats on a liquid surface; and which is mainly formed of powder-like microalgae which is referred to as blue-green algae. Therefore, the Microcystis is different from the biofilm according to the present invention.

Furthermore, Microcystis or red tide causes damage to fish and shellfish or the like which are bred by an aquaculturist due to inclusion of a toxic compound or its odor. Research and development related to suppressing the occurrence of the Microcystis or the red tide have been conducted. In addition, research and development related to a method of efficiently removing the Microcystis or the red tide from the liquid surface when the Microcystis or the red tide occurs have been conducted. However, there is currently no efficient method. Particularly, when Microcystis or red tide occurs, the Microcystis or the red tide in the case of appearing on a liquid surface is not localized only to the liquid surface. The Microcystis or the red tide exists on the liquid surface in the largest amount and exists while gradually decreasing as water depth becomes deeper (Non-Patent Document 1). Accordingly, there is no efficient collecting method of the Microcystis or the red tide due to difficulty in the collection thereof because of its powdery shape and existence of many algal bodies in places other than those on the liquid surface. Furthermore, even if the Microcystis or the red tide could have been collected using an expensive device such as a centrifugal separator or the like and a large amount of electric energy, the water content ratio of the collected substance would have been greater than or equal to 90%, which is extremely high (Non-Patent Document 2).

In regards to culturing of Botryococcus, Non-Patent Documents 3 and 4 show that Botryococcus exhibits floating properties on a liquid surface when Botryococcus accumulates oil. In addition, Patent Document 1 discloses in the Abstract that Botryococcus floats on a liquid surface. However, in Patent Document 1, Botryococcus is cultured on a surface of a hygroscopic cloth based on a phenomenon that the accumulation rate of hydrocarbon compounds is improved through direct contact between carbon dioxide and algae, but there is no disclosure in Examples in Patent Document 1 that Botryococcus directly floats on a liquid surface.

Non-Patent Document 5 discloses Botryococcus sp. UTEX2629 strains as Botryococcus sudeticus. However, the strains have a greater specific gravity than that of Botryococcus braunii and there is no disclosure relating to the floating thereof on a liquid surface.

As described above, there is no disclosure of specific modes, in which Botryococcus actually floats on a liquid surface, in the related art while there is a suggestion that Botryococcus floats on a liquid surface. Needless to say, there is no disclosure of formation of a biofilm on a liquid surface.

### Related Art

### Patent Document

[Patent Document 1] US-A-2009-0087889

### Non-Patent Document

[Non-Patent Document 1] Ebara engineering review, pp. 32, No. 217 (2007-10)
[Non-Patent Document 2] NEDO Press Release, March 17, 2010, Succeeds in Extracting Green Crude Oil from Blue-Green Algae, Journal of the Agricultural engineering Society, pp. 45, Vol. 56, 1988
[Non-Patent Document 3] The Ecology of Cyanobacteria, Their Diversity in Time and Space, B. A. Whitton & M. Potts, Eds, Kluwer Academic (1999), pp. 160
[Non-Patent Document 4] Oceanological Studies, 1998, Vol. 27, No. 1, Publisher: Index Copernicus p. 17
[Non-Patent Document 5] Melis et al., J apply Phycol (2010)

### Summary of Invention

### Problems that the Invention is to Solve

The present inventors have proposed a culturing method in which a uniform suspended solution of microalgae is prepared through a suspension treatment of microalgae and culturing is started in this state. In the culturing method, microalgae are sunk to the bottom surface, and a few days after culturing is continuously performed, a film-like structure or a three-dimensional structure, which is derived from microalgae on a liquid surface, is formed. In many cases, the culturing of microalgae is generally performed while enlarging the culture scale in several stages. The present invention is a proposal for overcoming a problem of a decrease in efficiency of the suspension treatment of microalgae due to an enlarged culture scale to some extent. An object of the present invention is to provide a method of culturing microalgae capable of reducing the production cost of biomass derived from microalgae.

Culturing microorganisms such as microalgae and extracting biomass such as biodiesel or hydrocarbon compounds from the cultured microorganisms have been attracting great attention. However, production thereof has not yet been carried out on a commercial scale. One of the reasons for this is that the production cost thereof is too high compared to energy resources derived from fossils which can be obtained from oil fields or the like. Primary causes of the increased production cost are that the cost for collecting microalgae with small size which are dispersed in liquid, from the liquid is high; a large area is generally required for producing biomass and there is no method of uniformly introducing carbon dioxide to such a large area at a low cost; the installation cost is high because of extremely long-distance piping for supplying carbon dioxide; the production output of biomass of algae per unit of light receiving surface is small; the apparatus cost and operation cost for stirring a liquid medium in which algae are dispersed are high; the water content ratio of the collected substance is high and energy is required for a dehydration process during an oil extraction process subsequent to the collecting; and handling of a large amount of the liquid medium is difficult and is a process that requires high energy usage.

In addition, another object of the present invention is to provide a biofilm formed on a liquid surface through the culturing method according to the present invention; biomass and oil obtained from the biofilm; and a method of producing biomass derived from microalgae.

The present inventors have formed a biofilm as a film-like structure or a three-dimensional structure which is formed of microalgae, on a liquid surface such that microalgae are subjected to a suspension treatment; a solution containing the microalgae is introduced to a culture solution after being uniformized; and once the microalgae are deposited on the bottom surface of the culture solution in a culture container, culturing is continuously performed for several days. In this method, it is possible to form a uniform film-like structure formed of microalgae through the suspension treatment at least in an initial stage of the culturing. However, movement of microalgae to a bottom surface of a culture container or movement of the algae to a liquid surface from the bottom surface of the culture container inevitably requires some time.

In general, culturing of microalgae is not performed by culturing a large area all at once, but is performed while gradually increasing the size of a culture container (culture pond). In culturing in a stage in which the culture container is small, it is possible to perform suspension treatment of microalgae after collecting the microalgae on a liquid surface and to start the culturing after preparing a medium of a larger culture container and preparing a uniform solution of the microalgae. However, if the culture container becomes larger, it is impossible to ignore the time required for suspension treatment or introduction of a device therefor; and the amount of energy for maintenance and for producing and operating the device. Furthermore, it is necessary to uniformly mix a large amount of the solution and a culture solution for performing subsequent culturing. In both the methods, it is expected that the methods may be troublesome due to an increased number of processes and that the ratio of the amount of acquired energy to the amount of input energy may become smaller.

### Means for Solving the Problems

The present invention has been completed based on such knowledge in order to solve the above-described problems and has the following configuration.
[1] A method of culturing microalgae, comprising:
   culturing in a first stage in which microalgae obtained through a purification process are cultured in a culture solution within a first culture container to form a biofilm as a film-like structure or a three-dimensional structure which is formed of the microalgae on a liquid surface of the culture solution; and
   culturing in a second stage in which microalgae are cultured on a liquid surface using at least a part of the biofilm as a film-like structure or a three-dimensional structure which is formed on the liquid surface of the culture solution, as seed algae.
[2] The method of culturing microalgae according to [1],
   wherein the culturing in the second stage is performed by collecting a part of or the entirety of the biofilm as a film-like structure or a three-dimensional structure which is formed on the liquid surface in the culturing in the first stage, from the first culture container, transferring the collected biofilm into a second culture container, and using the biofilm as seed algae in a culture solution in the second culture container.
[3] The method of culturing microalgae according to [1] or [2],
   wherein the culturing in the second stage is performed by collecting a part of the biofilm as a film-like structure or a three-dimensional structure which is formed on the liquid surface in the culturing in the first stage, plural times using a substrate, transferring the collected biofilms into a plurality of second culture containers, and using the biofilms as seed algae in culture solutions in the respective plurality of second culture containers.
[4] The method of culturing microalgae according to [1],
   wherein the culturing in the second stage is performed by collecting a part of the biofilm as a film-like structure or a three-dimensional structure which is formed on the liquid surface in the culturing in the first stage, from the first culture container, and using a biofilm remaining in the first culture container, as seed algae, in the culture solution in the first culture container.
[5] The method of culturing microalgae according to any one of [1] to [4], further comprising:
   performing a division treatment, in which the biofilm as a film-like structure or a three-dimensional structure which is formed on the liquid surface in the culturing in the first stage is divided, between the culturing in the first stage and the culturing in the second stage.
[6] The method of culturing microalgae according to [5],
   wherein the division treatment is performed on a liquid surface where a region, in which a biofilm substantially exists, and a region, in which a biofilm does not substantially exist, coexist.
[7] The method of culturing microalgae according to [6],
   wherein the divided biofilm is moved to the region in which a biofilm does not substantially exist, through the division treatment.
[8] The method of culturing microalgae according to any one of [1] to [7],
   wherein the biofilm as a film-like structure or a three-dimensional structure which is formed on the liquid surface in the culturing in the first stage is collected using one sheet of a substrate which has a plurality of sites for collecting a biofilm.
[9] The method of culturing microalgae according to any one of [2], [3], and [5] to [8],
   wherein the area of the second culture container is larger than that of the first culture container.
[10] The method of culturing microalgae according to [9],
   wherein the first culture container, which has the biofilm as a film-like structure or a three-dimensional structure which is formed on the liquid surface after the culturing in the first stage, is allowed to sink in the second culture container to float the biofilm on the liquid surface of the second culture container and to transfer the biofilm to the second culture container.
[11] The method of culturing microalgae according to any one of [1] to [10],
   wherein when collecting a part of the biofilm as a film-like structure or a three-dimensional structure which is formed on the liquid surface in the culturing in the first stage from the first culture container, the biofilm corresponding to 25% to 75% of the area of the liquid surface is collected.
[12] The method of culturing microalgae according to any one of [1] to [11],
   wherein both the culturing in the first stage and the culturing in the second stage are stationary culture.
[13] The method of culturing microalgae according to any one of [1] to [12],
   wherein the biofilm as a film-like structure or a three-dimensional structure which is formed on the liquid surface in the culturing in the first stage is collected by being transferred to or deposited on the surface of a substrate.
[14] The method of culturing microalgae according to any one of [1] to [13],
   wherein the microalgae are green algae.
[15] The method of culturing microalgae according to any one of [1] to [14],
   wherein the microalgae are microorganisms containing oil.
[16] The method of culturing microalgae according to any one of [1] to [15],
   wherein the microalgae belongs to the genus Botryococcus (Botryococcus sp.).
[17] The method of culturing microalgae according to [16],
   wherein the microalgae belongs to Botryococcus sudeticus.
[18] The method of culturing microalgae according to [17],
   wherein the microalgae are Botryococcus sudeticus AVFF007 strains (deposition number of FERM BP-11420).
[19] A biofilm which is formed on a liquid surface through the method of culturing microalgae according to any one of [1] to [18].
[20] Biomass which is obtained from the biofilm according to [19].
[21] Oil which is obtained from the biofilm according to [19].
[22] A method of producing biomass derived from microalgae, comprising:
   the method of culturing microalgae according to any one of [1] to [18].

### Advantage of the Invention

According to the method of culturing microalgae according to the present invention, it is possible to perform subsequent culturing without preparing a suspended solution of microalgae. Furthermore, it is unnecessary to introduce the suspended solution of microalgae to the next culture container and to stir the entire medium existing in the culture container. Moreover, it is possible to substantially stir or disperse only a biofilm of microalgae, which is on a liquid surface, on the liquid surface to thereby efficiently perform culturing. Furthermore, it is possible to form a biofilm on the liquid surface, and thus, it is extremely easy to collect microalgae unlike in floating culture in the related art. That is, in the present invention, a biofilm formed of an aggregate of microalgae floats on a liquid surface in a stage of collecting microalgae and the biofilm is set as a subject to be collected. Therefore, it is unnecessary to collect microalgae from a large amount of medium unlike in the related art, and only the biofilm on the liquid surface and moisture contained in the biofilm are set to a subject to be collected. For this reason, it is possible to greatly reduce the collection cost of microalgae. In addition, it is unnecessary to handle a large amount of liquid medium and to use a large amount of water.

### Brief Description of Drawings

Fig. 1 is a view showing a basic configuration of a culturing method of the present invention.
Fig. 2 is a view showing a partial collection state of a biofilm on a liquid surface, in the culturing method of the present invention.
Fig. 3 is a view showing an example of a method of introducing the biofilm obtained through culturing in a first stage to a liquid surface of a medium in culturing in a second stage.
Fig. 4 is a view showing an example of a method of moving a biofilm as a film-like structure or a three-dimensional structure, which is formed on the liquid surface, to a different culture container.
Fig. 5 is a state in which a biofilm which is formed on a liquid surface and is formed of microalgae (film-like structure in Fig. 5) is collected using a second substrate (glass substrate).
Fig. 6 is a state of the biofilm deposited on the second substrate (glass substrate) after collecting the bioflm formed on the liquid surface of Fig. 5.
Fig. 7 is a composition of a CSiFF03 medium.
Fig. 8 is a composition of a CSiFF01 medium.
Fig. 9 is a view showing a relationship between the number of days of culturing in the second stage and the quantity of dry algal bodies of the biofilm formed on the liquid surface, in Example 1.
Fig. 10 is a view showing a result of the quantity of dry algal bodies of a biofilm formed on a liquid surface 21 days after the start of the culturing in the second stage, and a result of the quantity of dry algal bodies 0 days after the start of the culturing in the second stage, in each of Example 1 and Reference Example 1.
Fig. 11 is a composition of a CSi medium.
Fig. 12 is a view showing photographs of microalgae on a liquid surface 0 days, 4 days, 7 days, 11 days, and 13 days after the start of culturing in a second stage in Example 2.
Fig. 13 is a composition of a CSiFF04 medium.
Fig. 14 is a view showing a relationship between the water depth in the culturing in the second stage and the quantity of dry algal bodies of the biofilm formed on the liquid surface after performing the culturing in the second stage for 14 days.
Fig. 15 is a view showing a relationship between the ratio of biofilm remaining on a liquid surface after the culturing in the first stage, the quantity of dry algal bodies of the biofilm formed on the liquid surface after the culturing in the second stage, and the proliferation ratio of microalgae.
Fig. 16 is a view showing a result of the quantity of dry algal bodies of a biofilm formed on a liquid surface 4 days and 12 days after the start of the culturing in a second stage, with respect to culture containers which are denoted as "no partial harvest", "parent", and "child 1" to "child 3" and are prepared in Example 5.
Fig. 17 is a composition of a C medium.
Fig. 18 is a view showing a microscopic photograph (magnification of 40) of AVFF007 strains.
Fig. 19 is a base sequence (SEQ ID No: 1) of the AVFF007 strains used in BLAST analysis.
Fig. 20 is a systematic diagram of Botryococcus sudeticus AVFF007 strains of microalgae.
Fig. 21 is a view showing a relationship between "parents" and "children" in culturing from the start of the culturing to harvest.

### Description of Embodiments

Hereinafter, an embodiment of a method of culturing microalgae of the present invention will be described in detail.

The method of culturing microalgae of the present invention includes: culturing in a first stage in which microalgae obtained through a purification process are cultured in a culture solution within a first culture container to form a biofilm as a film-like structure or a three-dimensional structure which is formed of the microalgae on a liquid surface of the culture solution; and culturing in a second stage in which microalgae are cultured on a liquid surface using at least a part of the biofilm as a film-like structure or a three-dimensional structure which is formed on the liquid surface of the culture solution, as seed algae.

In the method of culturing microalgae of the present invention, microalgae capable of forming a biofilm on a liquid surface are moved on a new liquid surface or remain on the liquid surface on which the microalgae exist originally while maintaining at least a partial structure of the biofilm, and culturing is performed again so as to grow the biofilms on the liquid surfaces.

According to the present invention, it is possible to perform subsequent culturing without preparing a suspended solution of microalgae. Furthermore, it is unnecessary to introduce the suspended solution of microalgae to the next culture container and to stir the entire medium in the culture container. Moreover, it is possible to stir or disperse only a biofilm of microalgae on a liquid surface to thereby efficiently perform culturing. Furthermore, a biofilm formed on a liquid surface is set to a subject to be collected without collecting microalgae with an extremely small size which are dispersed in a liquid medium as in the related art, and therefore, it is easy to perform collection. Furthermore, in the case of floating culture in a liquid medium as in the related art, it is necessary to perform an operation, such as a centrifugal operation or an evaporation operation, which has low energy efficiency in order to remove moisture since there is a large amount of moisture remaining after the collection of the microalgae. However, in the method of the present invention, a biofilm which is in a form where microalgae are aggregated at high density is set to a subject to be collected, and therefore, it is possible to obtain a collected substance with a lower water content ratio compared to the former method.

In addition, in the method of the present invention, culturing is performed at a gas-liquid interface, and thus, it shows a low death rate of algal bodies due to drying.

It is preferable that in the method of culturing microalgae according to the present invention, the culturing in the second stage is performed as a first mode by collecting a part of or the entirety of a biofilm as a film-like structure or a three-dimensional structure which is formed on the liquid surface in the above-described culturing in the first stage, from the first culture container, transferring the collected biofilm into a second culture container, and using the biofilm as seed algae in a culture solution in the second culture container.

In the above-described first mode, it is more preferable to perform the culturing in the second stage by collecting a part of the biofilm as a film-like structure or a three-dimensional structure which is formed on the liquid surface in the culturing in the first stage plural times using a substrate, transferring the collected biofilms into a plurality of second culture containers, and using the biofilms as seed algae in the culture solutions in the respective plurality of second culture containers.

In the above-described first mode, it is preferable that the area of the second culture container is larger than that of the first culture container.

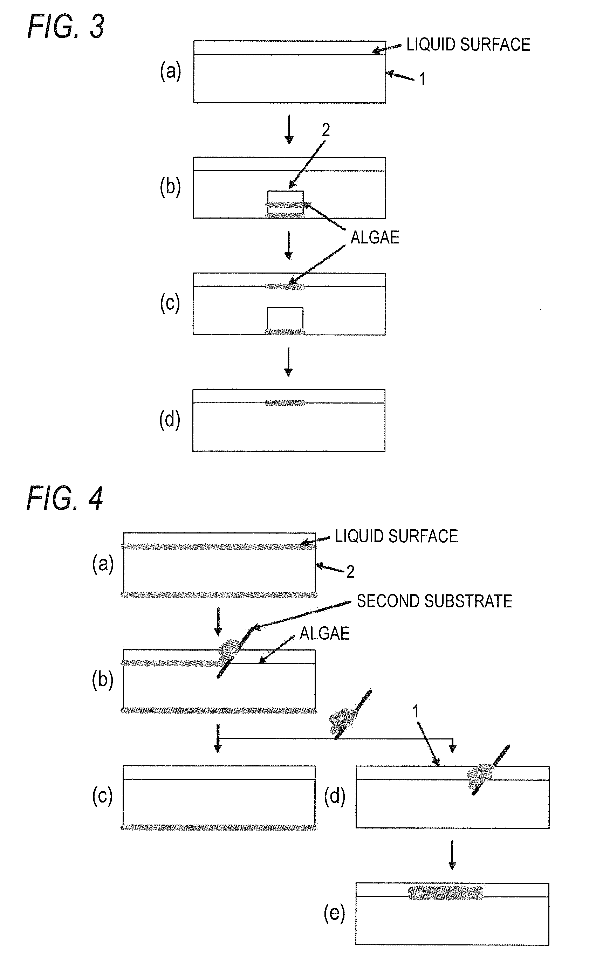

Furthermore, it is preferable that the first culture container, which has the biofilm as a film-like structure or a three-dimensional structure which is formed on the liquid surface, is allowed to sink in the second culture container after the culturing in the first stage, to be transferred into the second culture container by floating the above-described biofilm on a liquid surface of the second culture container. Specific examples thereof include a method shown in Fig. 3 to be described later, but the present invention is not limited thereto.

In addition, it is preferable that in the method of culturing microalgae according to the present invention, the culturing in the second stage is performed as a second mode by collecting a part of the biofilm as a film-like structure or a three-dimensional structure which is formed on the liquid surface in the above-described culturing in the first stage, from the first culture container, and using a biofilm remaining in the first culture container, as seed algae, in the culture solution in the first culture container.

It is preferable that the method of culturing microalgae of the present invention further includes performing a division treatment, in which the biofilm as a film-like structure or a three-dimensional structure which is formed on the liquid surface in the above-described culturing in the first stage is divided, between the culturing in the first stage and the culturing in the second stage.

Hereinafter, the details of the method of culturing microalgae of the present invention will be described.

### [Microalgae]

Microalgae in the present invention are typically microalgae capable of forming a biofilm on a liquid surface and have an ability of forming a biofilm on a liquid surface.

The microalgae referred to in the present invention indicate minute algae of which individual existence cannot be identified with the human naked eye. The classification of microalgae is not particularly limited as long as the microalgae have the ability of forming a biofilm on a liquid surface, and any of prokaryotes and eukaryotes may be used.

The microalgae are not particularly limited, and any microalgae can be appropriately selected depending on the purpose. Examples thereof include the division Cyanophyta, the division Glaucophyta, the division Rhodophyta, the division Chlorophyta, the division Cryptophyta, the division Haptophyta, the division Heterokontophyta, the division Dinophyta, the division Euglenophyta, and the division Chlorarachniophyta. These may be used alone or in a combination of two or more thereof. Among these, as the microalgae, the division Chlorophyta is preferable, and green algae are more preferable. As the microalgae, microorganisms containing oil are preferable, and the genus Botryococcus (Botryococcus sp.) is more preferable in terms of production of biomass.

The method of obtaining microalgae is not particularly limited, and any method can be appropriately selected depending on the purpose. Examples thereof include a method of collecting microalgae in nature, a method of using a commercially available product, and a method of obtaining microalgae from a storage institution or a depositary institution.

The biofilm referred to in the present invention is a film-like structure or a steric three-dimensional structure, to be described later, which is formed of microorganisms, and generally indicates a microorganism structure (microorganism aggregate or microorganism film) which is adhered to the surface of rock or plastic. Besides these, the biofilm is also regarded as a film-like structure or a steric three-dimensional structure, to be described later, which is formed of microorganisms existing on the surface such as a liquid surface having fluidity in the present invention. In general, a biofilm particularly in nature also contains debris or pieces of plants besides microorganisms, and the biofilm of the present invention may also contain them. Since it is impossible to avoid the mixing in of matter other than the target microorganisms in open environments such as the outdoors, a subject is not intentionally set to a sample containing them in the present invention. However, it is preferable that the biofilm does not contain impurities such as debris or pieces of plants in view of the efficiency of collecting microalgae. Ideally, it is more preferable that the biofilm is formed of only the microalgae according to the present invention, and a substance such as an intercellular matrix which is secreted during the proliferation of the microalgae. In addition, in the present invention, it is preferable that the biofilm is configured such that individual microalgae are adhered to each other directly or via a substance such as the intercellular matrix.

It is preferable that the microalgae capable of forming a biofilm on a liquid surface in the present invention are Botryococcus sudeticus microalgae. It is more preferable that the homology with base sequences of a partial region corresponding to Botryococcus sudeticus among base sequences encoding a gene region of 18S rRNA of the microalgae according to the present invention is 95.0% to 99.9%. The "partial region" referred to herein means a region having greater than or equal to 1000 base sequences. When testing the homology, use of every base sequence results in the highest reliability for the test of the homology. However, determining every base sequence is technically and financially difficult except for an extremely small number of species of organisms. In addition, only a specific portion (specifically, the vicinity of base sequences corresponding to base sequences of the AVFF007 strains which are set as a comparison target to be described later) of the base sequences of Botryococcus sudeticus has been reported. Furthermore, in general, it is known that attribution is possible if about 1000 base sequences are read. From the above, the homology has been tested through the comparison with the base sequences of a "partial region" in the present invention, and it is considered that the reliability is sufficiently high.

In addition, the microalgae according to the present invention preferably have a high proliferation rate on a liquid surface. Specifically, the proliferation rate in a logarithmic growth phase of microalgae on a liquid surface (that is, an average proliferation rate per day during the period of the logarithmic growth phase) is preferably higher than or equal to 0.1 g/m²/day with respect to dry weight, more preferably higher than or equal to 1 g/m²/day with respect to dry weight, still more preferably higher than or equal to 5 g/m²/day with respect to dry weight, and most preferably higher than or equal to 10 g/m²/day with respect to dry weight. The proliferation rate in the logarithmic growth phase of the microalgae on the liquid surface is generally lower than or equal to 1000 g/m²/day with respect to dry weight.

Particularly, the microalgae according to the present invention are more preferably Botryococcus sudeticus AVFF007 strains of microalgae (hereinafter, simply referred to as AVFF007 strains) from the viewpoints of good culturing on a liquid surface and good collection from the liquid surface; possession of a high proliferation rate; a high content of oil; little odor at least during culturing; and no generation of toxic substances being confirmed.

The AVFF007 strains as microalgae used in Examples of the present specification are internationally deposited to the Patent Organism Depositary of the National Institute of Technology and Evaluation (Room No. 120, 2-5-8 Kazusakamatari, Kisarazu-shi, Chiba-ken, Japan) dated September 28, 2011 with a deposition number of FERM BP-11420 [or to Patent Organism Depository of the National Institute of Advanced Industrial Science and Technology during international deposition (Central 6, 1-1, Higashi 1-chome, Tsukuba-shi, Ibaraki-ken, Japan)] under the Budapest Treaty.

The AVFF007 strains are new strains of fresh water microalgae which were isolated from a pond in Kyoto by the present inventors and belong to the genus Botryococcus and the species sudeticus.

Hereinafter, the method of isolating the microalgae (hereinafter, referred to as purification) and circumstances in which it has been determined that the AVFF007 strains of the microalgae are new strains will be described.

### (Purification of AVFF007 Strains of Microalgae)

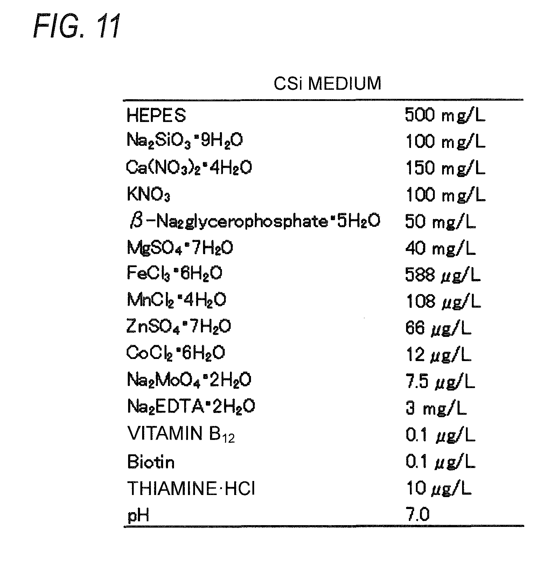

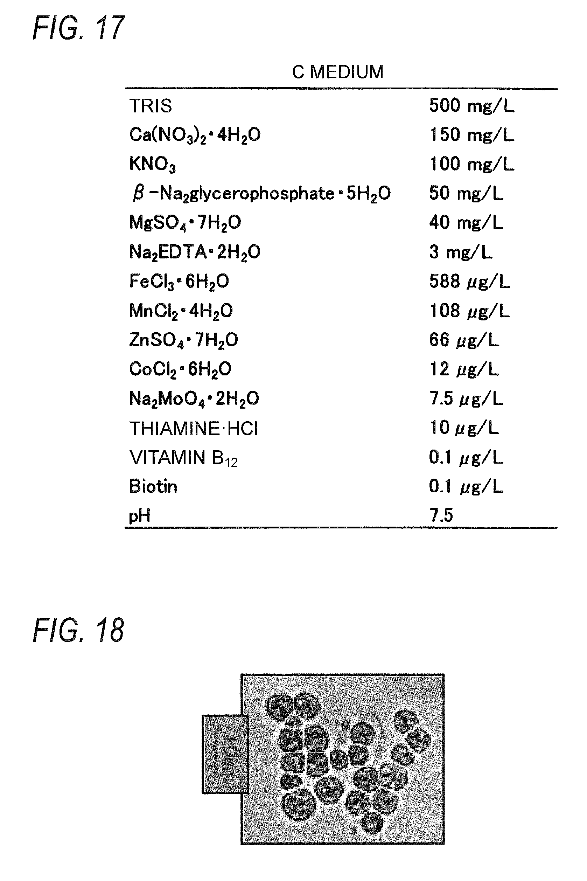

Natural fresh water was collected from a pond in Kyoto by putting it into a 5 mL tube for homogenizing (TM-655S, Tomy Seiko Co., Ltd.). 100 µL of the collected natural fresh water was added to a 24 hole plate (microorganism culture plate 1-8355-02, As One Corporation) into which 1.9 mL of a medium, in which a C medium shown in Fig. 17 and a CSi medium shown in Fig. 11 were mixed at 1:1 (volume ratio), was put. The plate was placed on a plant bioshelf for tissue culture (AV152261-12-2, Ikeda Scientific Co., Ltd.) and was cultured at 23°C under continuous irradiation with 4000 lux light. After approximately one month, a yellow aggregate was generated in wells of the 24 hole plate. The aggregate was observed using an optical microscope and it was confirmed that there are a large number of microorganisms.

1 g of agarose (Inviterogen, UltraPureTM Agarose) was weighed out, and 200 mL of a medium in which a C medium and a CSi medium were mixed at 1:1 (volume ratio) was put into a 500 mL conical flask. The medium was subjected to an autoclave treatment for 10 minutes at 121°C, and approximately 20 mL of the medium at a time was added to an Azunol Petri dish (1-8549-04, As One Corporation) in a clean bench before being cooled and hardened to produce agarose gel.

The solution containing the microalgae in the 24 hole plate was diluted, and the solution was made to adhere to a loop portion of a disposable stick (1-4633-12, As One Corporation) and was applied on the prepared agarose gel to prepare a Petri dish in which the microalgae were applied on the agarose gel.

The Petri dish was placed on a plant bioshelf for tissue culture and was cultured at 23°C under continuous irradiation with 4000 lux light. After approximately 2 weeks, a green colony appeared on the agarose gel. The colony was adhered to a tip end of a sterilized bamboo skewer (1-5980-01, As One Corporation), and then, was suspended in the wells of the 24 hole plate into which 2 mL of the medium, in which the C medium and the CSi medium were mixed at 1:1 (volume ratio), was put. The 24 hole plate containing microalgae prepared in this manner was placed on a plant bioshelf for tissue culture and was cultured at 23°C under continuous irradiation with 4000 lux light. After approximately 2 weeks, the aqueous solution in the wells exhibited a green color. A small amount of solution was collected from all of the wells, the microalgae were observed using an optical microscope, and it was concluded that purification had been performed in wells, in which it was considered that there was not only a single microalga.

The compositions of the C medium and the CSi medium were as shown in Figs. 17 and 11. For both the media, 900 mL of distilled water was subjected to an autoclave treatment for 10 minutes at 121°C and 100 mL of a C medium or a CSi medium at 10 times concentration was prepared. Then, the prepared medium was mixed with a solution which was sterilized using a filter having a pore size of 0.45 µm.

In addition, a view showing a microscopic photograph of AVFF007 strains at magnification of 40 is shown in Fig. 18.

### (Morphological Properties)

- All microalgae sink to the bottom surface if the microalgae are left for a while after performing a dispersion treatment.
- If the microalgae are cultured for a while, microalgae floating on a liquid surface appear. Accordingly, microalgae are divided into microalgae having sunk to the bottom surface and microalgae floating on the liquid surface. If further culturing is performed continuously, a film-like structure appears on the liquid surface. If the culturing is performed further, a three-dimensional structure appears.
- All the microalgae on the liquid surface and on the bottom surface have a spherical shape and have different size distributions.
- The microalgae have cohesiveness and form a large colony.
- The microalgae are green and the color thereof turns to yellow in accordance with the progress of the culturing.
- There is little odor in collected substances and during the culturing, but in some cases, there is a smell like fresh vegetables. There is a smell like sulfur when a solvent is removed from the collected substances.

### (Culturing Properties)

- Microalgae grow in fresh water whereas the proliferation in seawater becomes extremely slow. Only several % of seawater being mixed therein has an influence on the proliferation rate.
- During the cell proliferation, the cells proliferate through zoospores. Several zoospores to several tens of zoospores are generated from one cell.
- It is possible to perform photoautotrophic culture through photosynthesis.
- Nitrogen, phosphorus, potassium, calcium, magnesium, sulfur, manganese, and iron are essential for proliferation. In addition, inclusion of zinc, cobalt, molybdenum, and boron makes the proliferation favorable. Addition of vitamins also promotes the proliferation.

### (Physiological Properties)

- The growth temperature is less than or equal to 37°C. The higher the temperature is, the better the proliferating properties are.
- Microalgae mostly do not proliferate at 40°C, but can withstand at least for several hours under an environment of 40°C.
- The growth pH is 5 to 9. In some cases, the pH after the growth becomes higher than or equal to 8, for example, 10.5 depending on the kinds of media.
- If light or heat is provided, it is easy to generate carotenoid.
- Oil accumulates in a bacterial cell at 10 wt% to 30 wt% in terms of dry weight proportion.
- Algal bodies floating on a liquid surface have higher oil content than that of algal bodies having sunk to a bottom surface.
- Main components of the oil are hydrocarbon compounds and fatty acids. The fatty acids mainly include C16:0, C16:1, C18:1, and C18:2. The hydrocarbon compounds mainly include C17 and C21.
- AVFF007 strains dyed with Nile Red are observed by a fluorescence microscope. Then, it is confirmed that there is oil which was colored with the Nile Red as a bright fluorescent light-emitting region in algal bodies in a fluorescent visual field. The oil can accumulate in a wide region within an alga body cell.
- A culture solution containing the AVFF007 strainsis added dropwise to a slide glass, covered with a cover glass, and observed with a microscope. Then, oil-like oil droplets are released from the AVFF007 strains.
- Algal bodies floating on the liquid surface have a lower specific gravity than that of algal bodies having sunk to the bottom surface, but have a higher specific gravity than that of water.
- The amount of light at which it is possible to suitably perform proliferation is 200 µmol/m²/s to 800 µmol/m²/s. However, even at about 40 µmol/m²/s or at about 1500 µmol/m²/s, it is possible to perform the proliferation at a half proliferation rate compared to the suitable amount of light.

Identification of the AVFF007 strains was further performed through the following method.

### (Identification of AVFF007 Strains of Microalgae)

The culturing method of the AVFF007 strains was as follows. 50 mL of a CSi medium was introduced to a conical flask with a 100 mL capacity, 0.5 mL of an AVFF007 strain solution at a concentration of 1000 x 10⁴ cells/mL was added thereto, and shaking culture was performed under light irradiation for 14 days at 25°C.

In order to obtain a dry powder of the AVFF007 strains, centrifugal operation was performed on 40 mL of the medium containing the AVFF007 strains obtained as described above using a centrifuge (MX-300 (Tomy Seiko Co., Ltd.)) for 10 minutes at a centrifugal force of 6000 x g below 4°C. After removing a supernatant, the solid body was frozen together with the container using liquid nitrogen. Then, the total quantity of frozen solid body was transferred to a mortar which was chilled in advance using liquid nitrogen, and was ground using a pestle which was chilled in advance using liquid nitrogen.

The extraction of DNA from the microalgae was performed using DNeasy Plant Mini Kit (manufactured by Qiagen) according to the described manual. The purity and the amount of the extracted DNA were measured using e-spect (manufactured by Malcom Co., Ltd.). It was confirmed that the extracted DNA achieved the index of a purification degree which is A₂₆₀ₙₘ/A₂₈₀ₙₘ = 1.8 or greater, and about 5 ng/µL of DNA was taken.

There was no problem in the purification degree of the extracted DNA, and thus, a sample for PCR was prepared by diluting the DNA 10⁴ times in ultrapure water. An 18S rRNA gene region (rDNA region) was used as the sample for PCR. A cycle including 10 seconds at 98°C, 50 seconds at 60°C, and 10 seconds at 72°C was performed 30 times for the PCR using a GeneAmp PCR System 9700 (manufactured by Applied Biosystems). An enzyme used herein was Prime Star Max (manufactured by Takara Bio Inc.). It was confirmed through 1% agarose electrophoresis that the obtained PCR product was a single band.

The purification of the PCR product was performed using a PCR purification kit (manufactured by Qiagen). The method was carried out according to the method described in the manual. In order to check the purification degree and whether the PCR reaction was sufficiently performed, the purity and the amount of the DNA were measured using e-spect. It was determined that there was no problem since the measured purification degree was A₂₆₀ₙₘ/A₂₈₀ₙₘ = 1.8 or greater.

Next, the purified substance was used as a template and a cycle sequence was performed using a BigDye Terminator v3.1 Cycle Sequencing kit (manufactured by Applied Biosystems). The manual was referred to for the conditions. The base sequence of the obtained reactant was decoded using ABI PRISM 3100-Avant Genetic Analyzer (manufactured by Applied Biosystems).

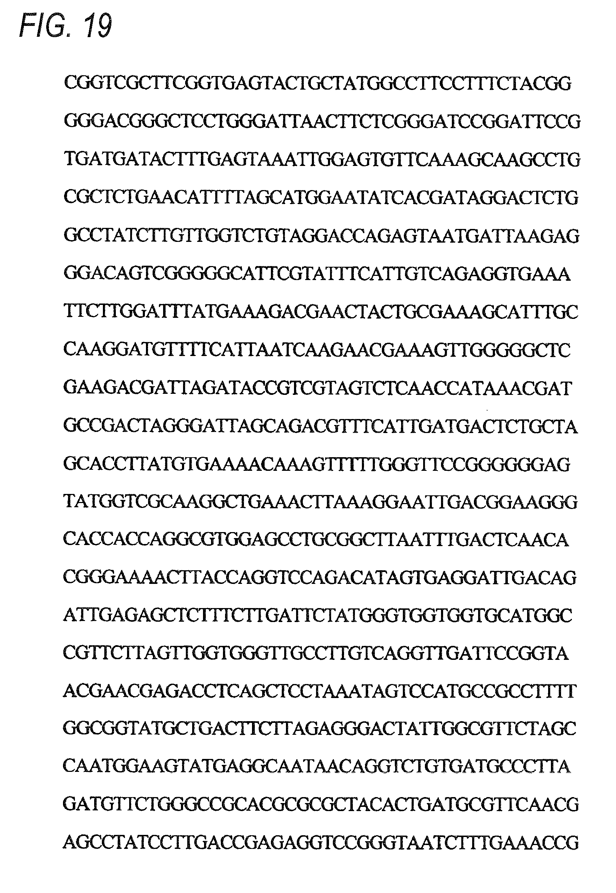

Homology analysis was performed using a basic local alignment search tool (BLAST). The method thereof was as follows. BLAST searching for the above-described sequence was conducted on the whole base sequence information in the data of the National Center for Biotechnology Information (NCBI). A species of an organism having the highest homology was regarded as a closely related species of the AVFF007 strains. Only the base sequence (1111 base, SEQ ID No: 1) which was set as a comparison target is shown in Fig. 19. Specifically, several bases at both the ends of the decoded base sequence were not set as a comparison target for the BLAST analysis, and thus are not shown in Fig. 19. The upper left of the base sequence shown in Fig. 19 is a 5'-terminal and the lower right thereof is a 3'-terminal.

As a result of the homology analysis, the above-described sequence had the homology (that is, 99% homology) to a 1109 base on the AVFF007 strain side among a Botryococcus sp. UTEX2629 strain side and 1118 bases on the Botryococcus sp. UTEX2629 strain side. Accordingly, the AVFF007 strains were classified as microalgae closely related to the Botryococcus sp. UTEX2629 strain.

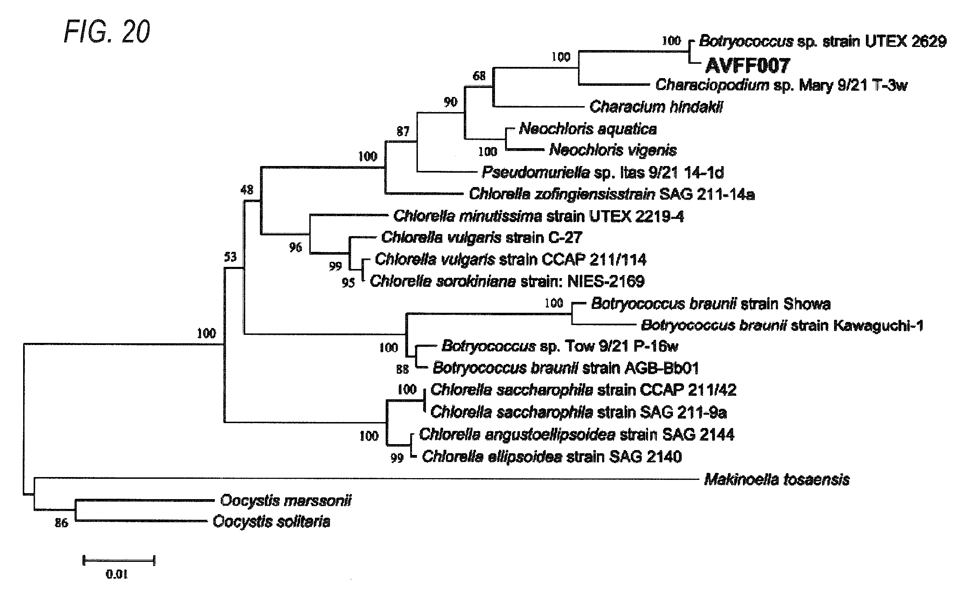

The systematic diagram obtained from the results of the above-described analysis is shown in Fig. 20. The AVFF007 strains are microorganisms which are also closely related to Characiopodium sp. Mary 9/21 T-3w, and there is a possibility that the name of the AVFF007 strains may be changed to the genus Characiopodium. In a case where the name of the Botryococcus sudeticus is changed, similarly, it is regarded in the present invention that the name of the AVFF007 strains is also changed. In addition, the same measure applies to a case where the name of the AVFF007 strain is changed to other names apart from the genus Characiopodium.

### (Density Measurement of AVFF007 Strains of Microalgae)

Cesium chloride was dissolved in a KOH solution (pH: 7.5) of 10 mM ethylenediaminetetraacetic acid (EDTA, ethylenediamine-N,N,N',N'-tetraacetic acid) and 5 mM HEPES (4-(2-hydroxyethyl)-1-piperazineethanesulfonic acid) to prepare solutions with a cesium chloride concentration of 35% to 105% (w/v) in steps of 10% in a cesium chloride concentration. Then, a concentration gradient was prepared such that the concentration became lower from a tip end portion of a Polyallomer tube (manufactured by Hitachi Koki Co., Ltd.) toward a liquid surface portion thereof.

5 x 10⁶ cells/mL of AVFF007 strains were applied on the upper surface of the tube and centrifugal treatment was performed using a centrifuge for 30 minutes at a centrifugal force of 20000 x g at 4°C.

The cell density of algal bodies floating on the liquid surface was 1.26 g/mL, which is less than 1.34 g/mL which is the cell density of the Botryococcus sp. UTEX2629 strains disclosed in Non-Patent Document 3.

From the above, it was determined that the algal bodies are not completely the same as the Botryococcus sp. UTEX2629 strains in view of the density and are microalgae close to the Botryococcus sp. UTEX2629 strains.

Furthermore, the fact that the algal bodies are microalgae closely related to the Botryococcus sp. UTEX2629 strains and they have almost the same form as each other can also be understood by comparing Fig. 3 of NPL 5 and the view showing the microscopic photograph of the AVFF007 strains shown in Fig. 18 of the present invention.

According to the culturing method of the present invention, it is considered that it is possible to form a biofilm on a liquid surface even with microalgae other than the above-described AVFF007 strains by setting favorable culture conditions.

### [Method of Present Invention]

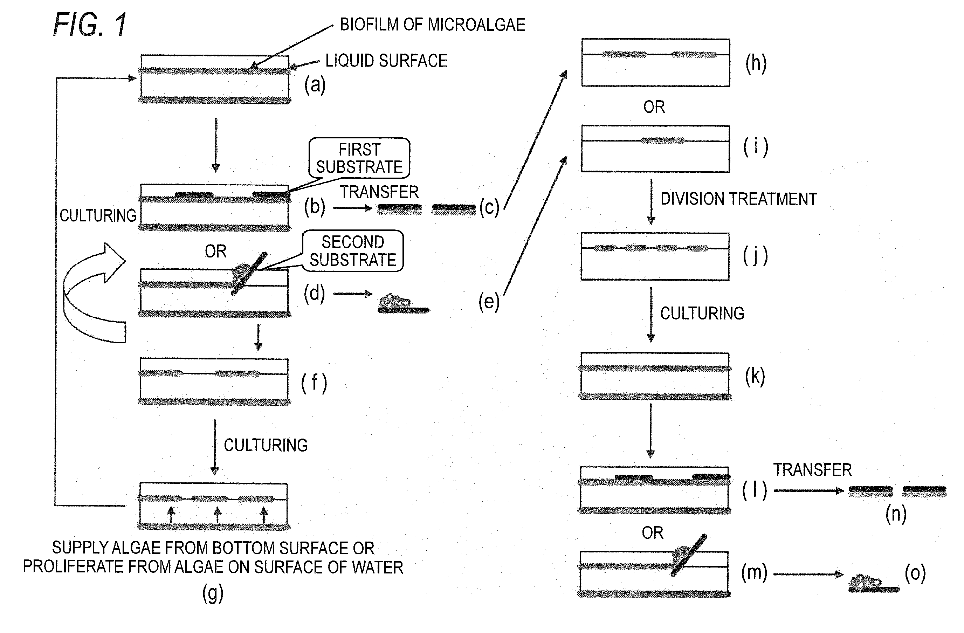

A basic structure of the culturing method of the present invention is shown in Fig. 1. The schematic view is used for describing the present invention, and therefore, description thereof is simplified in some sections.

First, culturing in a first stage will be described. As shown in (a) of Fig. 1, a biofilm as a film-like structure or a three-dimensional structure which is formed of microalgae is formed on a liquid surface through liquid surface-floating culture. As the forming method thereof, a suspended solution or a dispersed solution of microalgae is prepared and the microalgae sink to the bottom surface in several seconds to several tens of minutes depending on the kinds of microalgae by being set in a stationary state. In this state, a biofilm formed of the microalgae is formed on the liquid surface through culturing the microalgae for a while. The microalgae used in the culturing in the first stage may be prepared in this manner or prepared from the state of (g) of Fig. 1 or the state of (j) of Fig. 1 as will be described later. That is, the microalgae used in the culturing in the first stage may be prepared from a biofilm of microalgae floating on a liquid surface.

In the method of culturing microalgae of the present invention, culturing in a second stage is performed using at least one of a collected biofilm or a biofilm remaining after collection, as seed algae, by collecting at least a part of the biofilm as a film-like structure or a three-dimensional structure which is formed on the liquid surface through the above-described culturing in the first stage.

There are two methods of collecting microalgae as a biofilm on a liquid surface using a substrate, including a method of using a first substrate and a method of using a second substrate.

The method of collecting microalgae on a liquid surface using a first substrate is as follows. That is, the biofilm is transferred to the surface of the first substrate ((c) of Fig. 1) by bringing the film-like structure or the three-dimensional structure, which is formed of microalgae on the liquid surface, into contact with the first substrate as shown in (b) of Fig. 1. It is possible to collect the algal bodies by peeling off the transferred biofilm from the first substrate. The first substrate may be brought into contact with theentire liquid surface of the culture container, or may be brought into contact with a part of the liquid surface. Otherwise, whole contact and the partial contact may be repeated plural times. The collected amount of the microalgae on the liquid surface is increased by performing the contact plural times in this manner. In the above description, it is possible to transfer the film-like structure so as to be superposed on the surface of the first substrate. The film-like structure can be favorably used as the structure of the biofilm which becomes a subject of the method since it has good transfer efficiency.

The method of collecting microalgae on a liquid surface using a second substrate is as follows. That is, microalgae on the liquid surface is scraped off and collected using the second substrate as shown in (d) of Fig. 1 with respect to the film-like structure or the three-dimensional structure, which is formed of microalgae on the liquid surface. In the drawing, movement of the substrate from the right side to the left side is shown as an image. The movement direction of the second substrate may be reversed or the collection may be performed by being divided into plural times. When the collection is performed by being divided into plural times, a second substrate may be used in a state in which microalgae are adhered thereto, or a second substrate, from which microalgae are entirely or partially removed from the surface of the second substrate, may be used. In addition, only one sheet of the second substrate is drawn in Fig. 1, but plural sheets of the second substrates may be used at the same time. It is possible to freely set the angle, the size and the speed of the second substrate depending on the purpose thereof.

The collecting method using the second substrate can be more favorably used as the collecting method using a substrate in terms of the attachability/detachability of a biofilm from a substrate, and in general, because of the small area of the substrate.

In the present invention, it is possible to partially leave microalgae on the liquid surface during the collection. The proportion of the microalgae to be left can be arbitrarily determined in accordance with the culturing state.

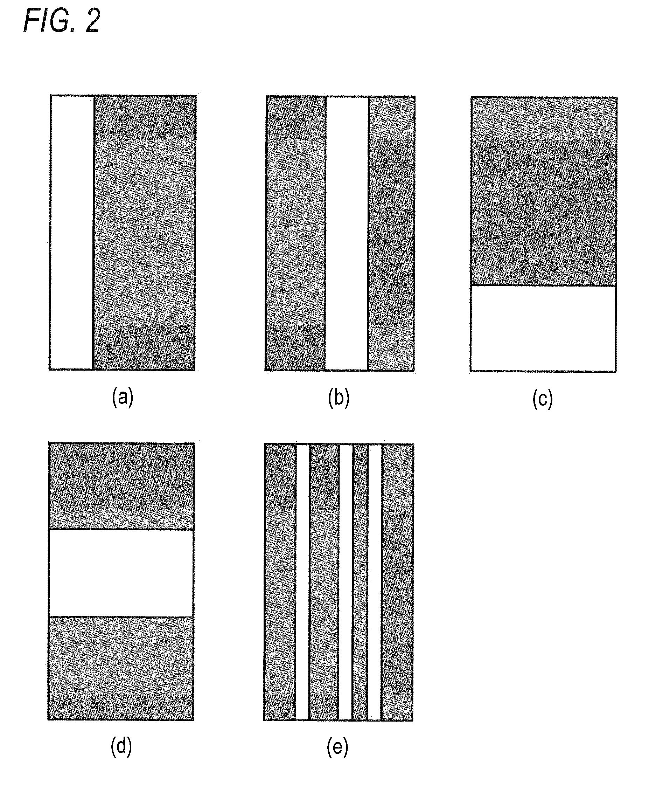

As the partial collecting method, a method of collecting a portion in which the side surface of a culture container and the liquid surface come into contact with each other as in (a) or (c) of Fig. 2 or a method of collecting a region between the side surface of a culture container and the side surface of the culture container as in (b) or (d) of Fig. 2 can be performed, when seen from above in Fig. 2 can be performed. In Fig. 2, white portion is a portion in which microalgae as a biofilm are collected (harvested), and the colored portion is a portion in which microalgae as a biofilm remain. In a case where a division treatment to be described later is not performed, collection according to (b) of Fig. 2 is most preferable. This is because a biofilm is at least partially destroyed by the collecting operation using a substrate and pieces of the biofilm are distributed also in a region where there is no biofilm, and therefore, there is an increased possibility that proliferation can be favorably performed.

One structure formed of microalgae on a liquid surface of a culture container may be formed as a region where there are microalgae on the liquid surface after the partial collection as shown in (a) or (c) of Fig. 2, or one region where there are no microalgae on the liquid surface may be formed. In addition, a plurality of structures formed of microalgae on the liquid surface and one region where there are no microalgae on the liquid surface may be formed as shown in (b) or (d) of Fig. 2. Furthermore, a plurality of structures formed of microalgae on the liquid surface and a plurality of regions where there are no microalgae on the liquid surface may be formed as shown in (e) of Fig. 2, or one structure formed of microalgae on the liquid surface and a plurality of regions where there are no microalgae on the liquid surface may be formed. The description is made on the assumption that the biofilm structure of microalgae remaining on the liquid surface does not move during or immediately after the collecting process.

After a portion of microalgae which is harvested according to (c) or (e) of Fig. 1 is collected from a substrate, a biomass extraction process may be performed or culturing may be started in a state where the collected microalgae are made to float on a liquid surface of a new culture container. That is, the process from (c) to (h) of Fig. 1 and the process from (e) to (i) of Fig. 1 correspond thereto.

Although the method of floating a film-like structure or a three-dimensional structure of microalgae on a liquid surface is not shown in Fig. 1, a method shown in Fig. 3 may be used. That is, the method is as follows. A culture solution in which microalgae can proliferate is put into a second culture container 1 (hereinafter, simply referred to as culture container 1) as in (a) of Fig. 3; and a first culture container 2 with microalgae (hereinafter, simply referred to as culture container 2), of which the size is smaller than that of the culture container 1 is immersed in the culture container 1. Then, many of the microalgae on a liquid surface within the culture container 2 rise to the top of a liquid surface formed in the culture container 1. Then, the culture container 2 is removed from the culture container 1 to continue culturing.

Although it is illustrated in the drawing such that there are no microalgae in the culture container 1, there may be a small amount of microalgae on the liquid surface, a bottom surface, and a side surface. In addition, the culture container 2 is set to be removed from the culture container 1, but culturing may be continued without removing the culture container 2 from the culture container 1. In addition, since it is necessary to put a culture container into another culture container, it is preferable that the size of the culture container 1 is greater than that of the culture container 2. Furthermore, when the height of the culture container 2 is higher than the water depth of the culture container 1, it is difficult to float the film-like structure or the three-dimensional structure of the microalgae on the liquid surface of the culture container 1. Therefore, it is preferable that the height of the culture container 2 is lower than the water depth of the culture container 1. The culture container 2 can also be immersed after the water depth of the culture container 1 is made to be deep by inclining the culture container 1. Even in this case, it is preferable that the height of the culture container 2 is lower than the water depth of the culture container 1 being inclined.

When removing the culture container 2 from the state of (c) of Fig. 3, it is preferable that the culture container 2 is pulled out from the culture container 1 after the culture container 2 is shifted in a horizontal direction. This is because if the location is not moved, there is a possibility that a part or the entirety of the biofilm of microalgae on the liquid surface formed in the culture container 1 is collected again using the culture container 2. When the culture container 2 containing microalgae is immersed in the culture container 1 as shown in Fig. 3, in many cases, the biofilm on the liquid surface in the culture container 2 rises to the top of the liquid surface formed in the culture container 1 in the vicinity of the immersion site in a vertical direction, in a state where there is relatively less destruction to the structure. In addition, it is possible to favorably remove the culture container 2 while horizontally moving the biofilm of microalgae on the liquid surface of the culture container 1 in (c) of Fig. 3. The reason for doing this is the same as that described above.

A method shown in Fig. 4 can be performed as the method of moving the film-like structure or the three-dimensional structure of microalgae to a different culture container. The method is a method in which a biofilm on a liquid surface is collected using a second substrate after forming the biofilm of microalgae on the liquid surface as shown in (a) of Fig. 4, and the microalgae are moved to the culture container 1 as shown in (d) to (e) of Fig. 1. It is possible to use, for example, a cell scraper or the like for an operation of peeling off microalgae from the second substrate and transferring the microalgae to the liquid surface of the culture container 1. That is, microalgae are peeled off from the second substrate using the cell scraper and are moved to the top of the liquid surface of the culture container 1. The second substrate is used in the schematic view, but it is possible to perform the same operation using the first substrate. In addition, as shown in (e) of Fig. 4, in many cases, many of the microalgae remain on the liquid surface through this operation.

Furthermore, a treatment (hereinafter, also referred to as division treatment) of dividing the structure formed of microalgae which has formed through the above-described methods may be performed. Due to the structure of the biofilm being partially destroyed by performing the division treatment, the number of microalgae capable of coming into contact with the medium increases. Therefore, it is possible to expect improvement in the proliferation rate of microalgae and to reduce the size of a region of a liquid surface where there are no microalgae, and therefore, it is possible to effectively utilize the liquid surface. It is necessary to perform a stirring operation with respect to the entire culture solution when performing dispersion culture. During the division treatment of the present invention, only the stirring operation on the liquid surface is required. Therefore, it is possible to reduce energy consumption or the time for the stirring and to simplify the device compared to the former case.

Any method can be used as the method of dividing a biofilm, and examples thereof include a method of imparting vibration to a liquid surface, such as a method of utilizing a wave on a liquid surface, a method of utilizing wind, a method of utilizing a water flow, and a method of utilizing a solid material. The method of utilizing a solid material refers to a treatment in which a structure formed of microalgae on a liquid surface is split using, for example, a tool such as a rod or a screw.

The division treatment is a treatment in which the state of (h) or (i) of Fig. 1 enters the state of (j), and more specifically, it is possible to perform the division treatment in the state of Fig. 2, the state of (c) or (d) of Fig. 3, and the state of (e) of Fig. 4.

After the division treatment, culturing in a second stage is performed in the state of (j) of Fig. 1 in which the divided biofilm is floating on the liquid surface, and as shown in (k) of Fig. 1, it is possible to form a biofilm formed of microalgae again on the liquid surface. Then, it is possible to collect the microalgae on the liquid surface as shown in (1) or (m) of Fig. 1. The collected substance can be used for extracting biomass or used as seed algae for subsequent culturing.

The culturing using the collected substance (harvested substance) of a biofilm is shown only once (one cycle). However, a required number of the processes can be performed any number of times (any number of cycles). That is, the process of returning to (a) again after (a), (b), (d), (f), and (g) of Fig. 1 can be performed any number of times. In addition, the shapes and the sizes of the culture containers are the same as each other in the drawing, but are not limited thereto. It is preferable that the sizes of the culture containers, that is, the surface areas of the liquid surfaces are gradually increased in view of improvement in the harvested amount of biomass of microalgae and improvement in the proliferation rate of microalgae. In the present invention, the processes of (b) to (c) and further to (h) of Fig. 1 are denoted as collection, and for example, the process of (1) to (n) or (m) to (o) of Fig. 1 is also denoted as collection. That is, both in the case of using a collected substance for subsequent culturing and the collecting during a process of extracting biomass from the collected substance are denoted as collection. Particularly, in some cases, the latter case is denoted as final collection in the present specification.

When finishing culturing or in culturing using a plurality of culture containers, microalgae on a liquid surface may be partially collected from a culture container, which is finally used. However, it is preferable to collect the entire quantity thereof. This is because the collected amount of the microalgae has increased.

The process of culturing can enter the state of (a) in Fig. 1 again from the state of (f) in Fig. 1 through the state of (g) in Fig. 1. At this time, use of one culture container is shown in the schematic view. However, it is also possible to change the culture container. For example, in a case where culturing starts in the state of (h) or (i) of Fig. 1, the film-like structure or the three-dimensional structure of microalgae on the liquid surface can be moved to another culture container, for example, a culture container with a larger size to continue the culturing.

In a case where microalgae are continuously cultured using a single culture container in the culturing in the first stage and the culturing in the second stage, movement of water or a medium is minimized, and therefore, it is possible to reduce the cost and the labor hours required for culturing. Even in a case where other culture containers are used in the culturing in the first stage and the culturing in the second stage, it is possible to obtain an equivalent effect as described above. That is, in a case of general floating culture, it is necessary to collect a part of a medium containing microalgae in a culture container, add a solution, which contains the microalgae, to the medium in the next culture container, for example, a culture container with a larger area, and to perform stirring in order to make the microalgae exist uniformly in the culture container. This is because it is easy to divide the structure of the microalgae, which floats on a liquid surface, on the liquid surface.

It is possible to divide a film-like structure or a three-dimensional structure formed on a liquid surface, on the liquid surface. Accordingly, it is possible to improve the culturing rate of microalgae by increasing the number of microalgae which can participate in proliferation. As a result, it is possible to improve the production efficiency of biomass using microalgae. It is possible to perform this method after a part of a film-like structure or a three-dimensional structure on a liquid surface is collected. However, an equivalent effect can be obtained even if the method is performed before the collection.

Even after microalgae on a liquid surface have been collected, microalgae remain on a bottom surface. If there are still nutrient components, which enable proliferation of microalgae, in the medium, it is possible to form a film-like structure or a three-dimensional structure of microalgae on the liquid surface by causing the microalgae to proliferate again from the bottom surface of the culture container. Accordingly, seed algae may not be introduced to the culture container every time. This process is shown in (g) of Fig. 1.

In the process from the state of (g) in Fig. 1 to the state of (a) in Fig. 1, there is a case where microalgae are supplied through proliferation from the liquid surface and a case where microalgae existing on the bottom surface are supplied. In any of the cases, seed algae may not be newly prepared in order to make the state of (a) in Fig. 1, and therefore, it is possible to reduce the cost and the labor hour required for culturing.

In the present invention, as shown in (a) of Fig. 1, there are usually microalgae on the bottom surface of the culture container. Microalgae are preferably in a stationary state in the present invention. However, it is possible to perform the same culturing as that in the present invention even if shaking culture is performed as long as microalgae are not strongly stirred. However, the number of algal bodies in a biofilm formed on a liquid surface is reduced, and therefore, the culturing in the stationary state is desirable. In addition, the culturing in the stationary state is more preferable since it is unnecessary to use energy or a device for stirring. The sinking of microalgae to the bottom surface means that the majority of microalgae sink to the bottom surface and does not mean a state in which microalgae completely disappear from the top of the liquid surface or in the middle of the water.

In regard to the timing of collecting microalgae on a liquid surface, it is possible to collect microalgae in a state where the liquid surface in a culture container is partially covered with the microalgae, but it is preferable to collect the microalgae after the entirety of the liquid surface in the culture container is covered with the microalgae in view of obtaining a large number of algal bodies of the microalgae. In addition, the collection may be performed after continuing culture for a while after the entirety of the liquid surface is covered with the microalgae. In the film-like structure and the three-dimensional structure, the quantity of microalgae per unit area of the latter is large, and therefore, it is more preferable to perform the collection in the state of the latter.

In addition, in the method of the present invention, culturing is performed at a gas-liquid interface, and thus, it shows a low death rate of algal bodies due to drying.

In the method of culturing microalgae of the present invention, it is preferable that a biofilm as a film-like structure or a three-dimensional structure which is formed on a liquid surface in culturing in a first stage is collected by being transferred to the surface of a substrate or by being deposited on the surface of the substrate.

Next, these collecting methods will be described.

### [Collection of Biofilm on Liquid Surface through Transfer using First Substrate]

A first substrate is gently inserted so as to be parallel to or almost parallel to a liquid surface in a culture container, and microalgae on the liquid surface are adhered to the first substrate. When performing the insertion, the first substrate is slightly obliquely inserted with respect to the liquid surface, and is then finally made parallel to the liquid surface. Then, it is possible to collect a large amount of biofilm on the liquid surface with a smaller number of times of transfer, which is preferable. It is possible to transfer the biofilm from the liquid surface of the culture container to the first substrate by gently lifting the first substrate to which the biofilm on the liquid surface is adhered.

### [Collection of Biofilm on Liquid Surface using Second Substrate]

As shown in (d) of Fig. 1, it is possible to scrape a biofilm on a liquid surface using a second substrate for collection. In the drawing, the substrate is moved from the right side to the left side. The movement direction of the second substrate may be reversed (that is, movement of the substrate from the left side to the right side of the drawing) or the collection may be performed plural times. When the collection is performed plural times, a second substrate may be used in a state in which a biofilm is adhered thereto; a substrate, from which biofilm is entirely or partially removed from the surface of the above-described first substrate, may be used as the second substrate; or a new substrate may be used. In addition, only one sheet of the second substrate is drawn in Fig. 1, but plural sheets of the second substrates may be used at the same time. Among these, resuming the collection using the same second substrate after removing the biofilm on the substrate using one sheet of the second substrate is preferable to be done as much as the strength of the second substrate allows in terms of the collection efficiency. In addition, it is possible to freely set the size of the second substrate, and the angle, the movement speed, or the like of the second substrate with respect to the liquid surface depending on the purpose thereof. (e) of Fig. 1 is a state in which the biofilm is collected on the second substrate.

Hereinafter, the collection using the second substrate will be described in more detail.

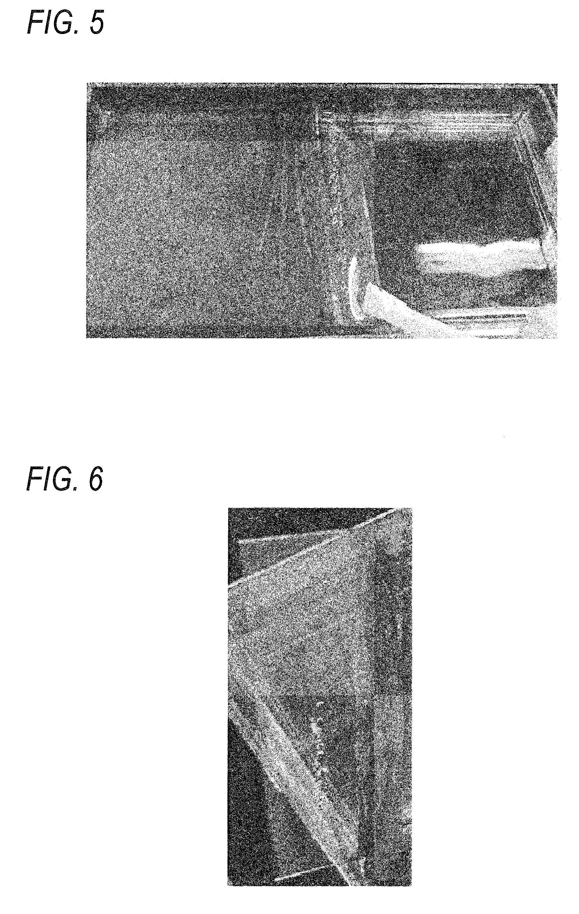

Fig. 5 is a state in which a film-like structure formed of microalgae floating on a liquid surface is collected using the second substrate (slide glass (76 mm x 26 mm) after forming a biofilm (film-like structure in the example of the drawing) on the liquid surface.

In Fig. 5, the biofilm is collected by obliquely inserting the long side of the slide glass into the biofilm on the liquid surface and advancing the slide glass in a left direction as it is, and by depositing the biofilm on the liquid surface on the slide glass. The left side of the drawing indicates a liquid surface on which the biofilm before being collected is formed and the right side of the drawing indicates a liquid surface after the biofilm is collected.

Fig. 6 shows a state of a biofilm deposited so as to be folded over the slide glass. The collection using the second substrate corresponds to a process from (d) to (e) or (m) to (o) of Fig. 1. In addition, it is possible to use substrates such as a nylon film other than a slide glass as the second substrate. In addition, the size of the substrate can be appropriately changed depending on the size of the culture container, but is preferably the same as that of a short side of the culture container. In addition, it is preferable that the culture container has a shape in which no gap can be created between the second substrate and the wall surface of the culture container. Preferred examples of such a shape include a rectangular type or a raceway type.

In regards to the timing of collecting a biofilm on a liquid surface or performing final collection, it is possible to collect the biofilm in a state in which the liquid surface in the culture container is partially covered with the biofilm, but it is preferable to collect the biofilm after the entirety of the liquid surface in the culture container is covered with the biofilm in view of obtaining a large number of algal bodies of microalgae. In addition, the collection may be performed after continuing culture for a while after the entirety of the liquid surface is covered with the biofilm.

Particularly, it is preferable to perform the collection or the final collection after a three-dimensional structure, to be described later, is formed on the liquid surface. This is particularly preferable in the final collection. In general, the three-dimensional structure is a structure which can be seen when proliferation of a film-like structure is further progressed. In the three-dimensional structure, a larger number of microalgae which can be collected and a lower water content ratio of the collected substance can be obtained compared to in the two-dimensional film-like structure.

(f) of Fig. 1 is a state after the biofilm on the liquid surface is collected. Microalgae are adhered to or deposited on the bottom surface of the culture container. In the schematic view of the present invention, there is illustration that the supply of the microalgae is performed also from the bottom surface to the top of the liquid surface. However, in reality, there are microalgae at a low concentration in the medium even at locations other than the liquid surface and the bottom surface, and microalgae can be supplied therefrom. Here, it is disclosed such a manner that the number of zoospores existing is excluded. Even in the state where the microalgae are supplied from the liquid surface and the bottom surface to the middle of the water, in the present invention, there is description that the microalgae are supplied from the bottom surface of the culture container to the top of the liquid surface. In addition, there are two cases for the supply of the microalgae from the bottom surface of the culture container to the top of the liquid surface, including a case in which the microalgae are moved to the top of the liquid surface without actually being accompanied by proliferation of the microalgae on the bottom surface and a case in which the microalgae proliferate while being moved from the bottom surface to the top of the liquid surface.

The culture container may be an open system or a closed system. However, it is more preferable that the culturing is performed in a closed system in view of improvement in the proliferation rate due to use of carbon dioxide at a high concentration, prevention of microorganisms other than target microorganisms, or debris from being mixed thereinto from the outside, prevention of a biofilm on the liquid surface from being destroyed or moved due to wind, and prevention of water from being evaporated.

In the present invention, as shown in the state of (a) of Fig. 1, the culturing method of culturing microalgae on the liquid surface is called liquid surface-floating culture. That is, a culturing method of culturing microalgae in only any one of the middle of the liquid or the bottom surface of the liquid or in both the middle of the liquid and the bottom surface of the liquid is not included in the liquid surface-floating culture.

The liquid surface in the present invention is typically a liquid surface of a liquid medium to be described later, and is generally an interface between the liquid medium and the air.

Furthermore, performing the liquid surface-floating culture in a stationary state as in (a) of Fig. 1 is called liquid surface-floating culture through stationary culture in the present invention.

According to an example of the embodiment of the present invention, it is possible to prepare a suspended solution including microalgae by suspending microalgae which are obtained through a purification process in a liquid medium containing an artificial medium. In addition, it is possible to form a film-like structure or a three-dimensional structure of microalgae on a liquid surface of a liquid medium and to partially or entirely collect the film-like structure or the three-dimensional structure by performing the culture in a culture apparatus. Moreover, it is possible to form a film-like structure or a three-dimensional structure of microalgae on a liquid surface by performing culture on the liquid surface again and to collect the proliferating microalgae from the entire culture tank or the liquid surface.

### [Purification Process]

The purification process referred to in the present invention is a process of isolating only target microalgae from a state in which various kinds of organisms, debris, and the like coexist and grow together with microalgae in a case of microalgae in a natural state. In some cases, such complete isolation is practically difficult, and therefore, in the present invention, it is defined that purification is completed as long as the target microalgae are primarily obtained.

The primary acquisition thereof in the present invention means a case in which microalgae other than a target could not be confirmed in several visual fields when observed with a microscope.

Examples of the purification method include a method of collecting a colony which is formed through culturing after developing a diluted suspended solution containing microorganisms on an agar medium, or a method of collecting microalgae one by one under a microscope.

### [Pre-culturing Process]

The pre-culturing process referred to in the present invention is a process of increasing the number of microalgae until the main culturing can be performed by causing stored microalgae to proliferate while microalgae obtained after the completion of the purification process is stored normally. Any well-known method can be used as the culturing method in the pre-culturing process. In addition, it is possible to perform the liquid surface-floating culture of the present invention. In addition, the pre-culturing process may be performed several times to cause the microalgae to proliferate until the microalgae reach a scale in which main culturing can be performed.

In addition, a culturing tank having a surface area of 1 cm² to 1 m² is generally used. It is possible to perform the culturing in either of an indoor place or an outdoor place, but it is preferable to perform the culturing indoors.

### [Main Culturing Process]

The main culturing process referred to in the present invention is a culturing process which is performed after the pre-culturing process is performed and immediately before the final collecting process is performed. The main culturing process may be performed plural times.

In addition, a culturing tank having a surface area greater than or equal to 100 cm² is generally used. It is possible to perform the culturing in either of an indoor place or an outdoor place, but it is preferable to perform the culturing outdoors.

The culturing in the first stage and the culturing in the second stage in the method of culturing microalgae of the present invention correspond to the main culturing process.

### [Seed Algae]

The seed algae in the method of culturing microalgae of the present invention refers to microalgae which are used when starting the above-described pre-culturing process or main culturing process, and become a base for proliferating microalgae in the pre-culturing process or the main culturing process. The main culturing process is not limited to a main culturing process immediately after the pre-culturing process, and also includes a second or subsequent main culturing process (for example, the above-described culturing in the second stage) which is further performed after the main culturing process. That is, the seed algae in the method of culturing microalgae of the present invention are not limited to microalgae obtained through the pre-culturing process, and also include microalgae (typically, a biofilm formed on a liquid surface through the main culturing process) obtained through the main culturing process.

### [Use of Microalgae]

Microalgae, which have been obtained through any well-known culturing method such as floating culture, liquid surface-floating culture, and adherent culture, may be used as microalgae used for performing the pre-culturing and the main culturing.

### [Suspension Treatment]

In the present invention, any treatment method for making aggregates of microalgae smaller aggregates or single microalgae can be included in the suspension treatment. Examples thereof include a gentle treatment such as a treatment of pipetting or shaking a solution of microalgae in a container by hand and a gentle treatment using a stirrer chip or a stirring rod; a strong treatment such as an ultrasonic treatment or a high speed-shaking treatment; and a method using a substance such as an enzyme decomposing an adhesion substance such as an intercellular matrix.

The contact area between the microalgae and the medium increases as the size of the aggregates of the microalgae become smaller compared to that of the aggregates of the microalgae having a larger size, when making the microalgae be smaller aggregates or single microalgae through the suspension treatment. As a result, in some cases, it is possible to improve the proliferation rate of the microalgae, which is preferable.

The ultrasonic treatment is a method of directly applying oscillatory waves, which have a high oscillation frequency and cannot be heard by the human ear, to a solution of microalgae or a container holding the solution of microalgae, and is a method in which the container is not necessarily a closed container and the solution containing the microalgae is not separated from the bottom surface of the container.

The high speed-shaking treatment refers to a treatment method in which a solution containing an aggregate of microalgae is added to a closed container for shaking such that an air layer can be formed and the entire closed container for shaking is shaken at a high speed. In the high speed-shaking treatment, the suspended solution layer is separated from or brought into contact with the inner wall of the closed container for shaking.

### [Suspended solution]

The suspended solution referred to in the present invention is a solution on which a suspension treatment is performed.

### [Suspended solution of Microalgae]

The suspended solution of microalgae refers to a solution which is obtained by performing a suspension treatment on microalgae. Microalgae which are cultured while floating in liquid may be used or microalgae which are cultured while adhering on a substrate may be used after being peeled off from the surface of the substrate. In addition, the microalgae which are obtained through the liquid surface-floating culture may be used. In addition, a mixture of microalgae derived from at least two or more of the culturing forms may be used. Examples thereof include a mixture of microalgae derived from the liquid surface-floating culture and microalgae on the bottom surface of the culture container during the liquid surface culture.

The suspension treatment may be performed for the pre-culturing and the main culturing in order to check the proliferation state of the microalgae. This is because it becomes easy to count the number of microalgae through the treatment.

### [Dispersion Treatment]

In the present invention, the dispersion treatment is a treatment method for making aggregates of microalgae smaller aggregates or single microalgae. For example, the dispersion treatment refers to a suspension treatment other than a gentle treatment such as a treatment of pipetting or shaking a solution of microalgae in a container by hand and a gentle treatment using a stirrer chip or a stirring rod. That is, the dispersion treatment includes a strong treatment such as an ultrasonic treatment or a high speed-shaking treatment and a method using a substance such as an enzyme decomposing an adhesion substance such as an intercellular matrix.

### [Dispersed Solution]

The dispersed solution referred to in the present invention is a solution in which a dispersion treatment such as an ultrasonic treatment or high speed-shaking treatment is performed, and is not a solution in which a gentle suspension treatment such as a treatment of pipetting or shaking a solution of microalgae in a container by hand and a gentle treatment using a stirrer chip or a stirring rod is performed.

### [Dispersed Solution of Microalgae]

The dispersed solution of microalgae refers to a solution which is obtained by performing dispersion treatment on the microalgae. Microalgae which are cultured while floating in liquid may be used, microalgae which are cultured while adhering on the substrate may be used after being peeled off from the surface of the substrate, or microalgae which are peeled off from the surface of the substrate at the same time as the suspension treatment may be used. In addition, the microalgae which are obtained through the liquid surface-floating culture may be used. In addition, a mixture of microalgae derived from at least two or more of the culturing forms may be used. Examples thereof include a mixture of microalgae derived from the liquid surface-floating culture and microalgae on the bottom surface of the culture container during the liquid surface culture.

In addition, the dispersion treatment may be performed for the pre-culturing and the main culturing in order to check the proliferation state of the microalgae. This is because it becomes easy to count the number of microalgae through the treatment.

### [Deposition]

The deposition referred to in the present invention refers to a state in which microalgae exist in or adhere to the vicinity of the bottom surface of the culture container or a state in which both the states coexist. The state in which microalgae exist in the vicinity thereof refers to a state in which the microalgae are easily moved from the substrate or the bottom surface of the culture container by slight movement of a liquid medium. In addition, the deposition includes a state of adhering.

### [Adhesion]

The adhesion referred to in the present invention refers to a state in which microalgae are directly adhered to the substrate or the bottom surface of the culture container and a state in which microalgae are adhered thereto to the extent that the microalgae are not peeled off from the substrate or the bottom surface of the culture container by slight movement of a liquid medium. In addition, in the present invention, in some cases, a state in which a biofilm (film-like structure or three-dimensional structure), which is formed of microalgae and is formed on a liquid surface of a liquid medium, floats on the liquid surface is described as a state in which the biofilm is adhered to the liquid surface.

### [Culturing Process]

The culturing process refers to a process for further increasing the amount of biofilm of microalgae existing on a liquid surface. When Fig. 1 is taken as an example, the culturing process refers to the process from (f) to (g) of Fig. 1, the process from (g) to (a) of Fig. 1, and the process from (j) to (k) of Fig. 1.

The culturing process also includes a process in which a biofilm is formed on a liquid surface, and then, the structure further proliferates. In general, a three-dimensional structure is formed after a film-like structure is formed, and the process is also included in the culturing process.

Shaking culture may be performed in the culturing process. This is because it is possible to form a film-like structure or a three-dimensional structure formed of microalgae on a liquid surface even through the shaking culture although the number of microalgae is smaller than that obtained through the stationary culture. However, it is preferable to perform the stationary culture in order to increase the number of proliferating biofilms formed of microalgae on the liquid surface. In addition, the stationary culture does not require any power for shaking, stirring, or the like, and requires minimum power energy or a minimum power generator unlike the floating culture in the related art, thereby reducing the cost greatly, which is preferable.

That is, in the method of culturing microalgae of the present invention, it is preferable to include the stationary culture of the microalgae capable of forming a biofilm on a liquid surface due to the above-described reasons.

### [Distribution State of Microalgae]

In the culturing of microalgae using the culturing method of the present invention, the distribution state of microalgae immediately before the collection of a biofilm is performed through the transfer using the first substrate, or using the second substrate has a characteristic in that the number of microalgae in the middle of liquid is smaller than the number of microalgae on the liquid surface or the number of microalgae deposited on the bottom surface of the culture container. In addition, in the distribution state at this time, it is preferable that the number of microalgae on the liquid surface is larger than the number of microalgae deposited on the bottom surface of the culture container. However, the present invention is not necessarily limited thereto. The number of microalgae referred to herein does not include zoospores which are generated from cells of the microalgae and which are difficult to observe using a microscope.

The number of microalgae in the middle of the liquid refers to the number of microalgae which are counted in the vicinity of the midpoint between the liquid surface and the bottom surface of the culture container, and refers to the number of microalgae per square centimeter. In addition, the number of microalgae in the vicinity of the top of the liquid surface or the bottom surface of the culture container refers to the number of microalgae per square centimeter. It is regarded that the number of microalgae can be replaced using a method which can determine the quantity of microalgae such as the weight, the dry weight, or the turbidity of microalgae.

### [Stationary Culture]

The stationary culture referred to in the present invention is a culturing method of culturing microalgae in a state of the microalgae not being intentionally moved. That is, for example, in some cases, a medium is circulated in accordance with local change in the medium temperature and microalgae are moved by the flow thereof. However, such a case is also called stationary culture in the present invention since the movement of the microalgae is not intentionally caused.

In the present invention, it is preferable to perform culture in the stationary state (that is, performing stationary culture) in all processes. This is done in order to prevent the biofilm (film-like structure or three-dimensional structure) formed of microalgae on a liquid surface from being destroyed. This is because there is a possibility that the collection efficiency using the second substrate may deteriorate and the microalgae may move to the middle of the liquid or to the bottom surface of the culture container when the biofilm formed of microalgae is destroyed. However, even if the culturing state is not stationary, it is possible to form the biofilm formed of microalgae on the liquid surface even when the number of microalgae is small. Therefore, the culturing in the stationary state is preferable in the present invention, but there is no restriction thereto. In addition, when performing a division treatment, it is possible to stir the liquid surface. The division treatment is a treatment which is performed in view of improving the proliferation rate of microalgae by uniformly distributing a biofilm of microalgae, which exists by being localized to a liquid surface, over a wide range of the liquid surface of a culture container as much as possible.

In the present invention, examples of methods of creating a state which is not stationary include a method of shaking the entire culture container, a method of performing stirring in the culture container using a stirring bar or a stirring rod such as a stirrer chip, a method of bubbling gas containing air or high-concentration carbon dioxide, and a method of making a suspended solution of microalgae flow.

In the method of culturing microalgae of the present invention, it is preferable that both the culturing in the first stage and the culturing in the second stage are stationary culture.

### [Adherent Culture]

The adherent culture referred to in the present invention is culturing in a state in which microalgae are adhered on the surface of a substrate or the wall surface of a culture container.

### [Floating Culture]

In the present invention, the culturing of microalgae in a medium is referred to as floating culture. In the present invention, a state in which microalgae are cultured while floating in liquid other than at the liquid surface, the side surface or the bottom surface of the culture container is called floating culture.

### [Liquid Surface-floating Culture]

In the present invention, the culturing method of culturing microalgae on a liquid surface is called liquid surface-floating culture. Even if microalgae exist on the bottom surface of a culture container, in the middle of a medium, or the like at the same time other than on the liquid surface, the culturing method is also called liquid surface-floating culture. In addition, microalgae being cultured by adhering on the liquid surface can also be considered as liquid surface-floating culture, and thus, in the present invention, the liquid surface-floating culture is considered as a kind of adherent culture.

In addition, in some cases, a phenomenon in which aggregates of microalgae seep out of the biofilm (film-like structure or three-dimensional structure) on the liquid surface to the middle of the liquid can be seen when the liquid surface-floating culture is performed in the present invention. In the present invention, the culturing in such a state is included in liquid surface-floating culture.

In the present invention, it is possible to perform the liquid surface-floating culture and to perform any one of the above-described floating culture and the adherent culture or both the floating culture and the adherent culture at the same time.

As described above, the names of the culturing methods are distinguished as described above depending on the place of culturing microalgae in the liquid medium in the present invention, but the methods can be performed at the same time as described above.

For example, a well-known method that can be used to culture microalgae belonging to the genus Botryococcus can be applied as the process of culturing microalgae of the present invention through the liquid surface-floating culture, the floating culture, and the adherent culture. For example, microalgae may be inoculated into a culture container such as conical flask into which a liquid medium is put, and be aeration-cultured under irradiation of light while being allowed to stand.

Next, the liquid medium which can be used in the liquid surface-floating culture, the floating culture, and the adherent culture will be described.

### [Medium (Liquid Medium)]

In the present invention, any well-known medium (liquid medium) can be used as long as it is possible to culture microalgae. It is desirable that the medium is selected depending on the kinds of microalgae to be cultured. For example, since the above-described AVFF007 strains are grown in fresh water, the medium is preferably fresh water. Examples of well-known media include an AF-6 medium, an Allen medium, a BBM medium, a C medium, a CA medium, a CAM medium, a CB medium, a CC medium, a CHU medium, a CSi medium, a CT medium, a CYT medium, a D medium, an ESM medium, an f/2 medium, a HUT medium, an M-11 medium, an MA medium, an MAF-6 medium, an MF medium, an MDM medium, an MG medium, an MGM medium, an MKM medium, an MNK medium, an MW medium, a P35 medium, a URO medium, a VT medium, a VTAC medium, a VTYT medium, a W medium, a WESM medium, a SW medium, and a SOT medium. Among these, freshwater media are an AF-6 medium, an Allen medium, a BBM medium, a C medium, a CA medium, a CAM medium, a CB medium, a CC medium, a CHU medium, a CSi medium, a CT medium, a CYT medium, a D medium, a HUT medium, an M-11 medium, an MA medium, an MAF-6 medium, an MDM medium, an MG medium, an MGM medium, an MW medium, a P35 medium, a URO medium, a VT medium, a VTAC medium, a VTYT medium, a W medium, an SW medium, and an SOT medium. As media for culturing the above-described AVFF007 strains, a C medium, a CSi medium, and a CHU medium, and a mixture of these media are preferable, and a C medium and a CSi medium, and a mixture of these media are more preferable.

The media may be subjected to ultraviolet ray sterilization, autoclave sterilization, and filter sterilization, or may not be sterilized.

In the present invention, it is preferable that a liquid medium contains calcium. That is, it is desirable to add calcium to the medium. This is because the proliferation rate of microalgae improves, and thus it is easy to form a biofilm (film-like structure or three-dimensional structure) on a liquid surface, when calcium is added to the medium. The concentration of calcium in a liquid medium is not particularly limited, but is preferably greater than or equal to 0.3 mM and more preferably greater than or equal to 0.5 mM.

The upper limit value of the concentration of calcium in a liquid medium is not particularly limited, but is generally less than or equal to 100 mM, preferably less than or equal to 50 mM, and more preferably less than or equal to 5 mM.

### [Water Depth]

The water depth of a liquid medium used in the present invention is not particularly limited, but it is preferable that the water depth is shallow. This is because the shallower the water depth, the smaller the amount of water used. In addition, this is because complication during handling, such as movement of water, or the amount of energy used also decreases. Furthermore, this is because the manufacturing cost of culture containers also decreases. However, it is necessary for a liquid medium to have a water depth to some extent since there is a possibility that the shallower the water depth, the more the nutriment increases per unit area. In the present invention, the water depth is preferably greater than or equal to 0.4 cm, more preferably 1.0 cm to 10 m, still more preferably 2.0 cm to 1 m, and particularly preferably 5.0 cm to 30 cm. When the water depth is greater than or equal to 0.4 cm, it is possible to form a biofilm. When the water depth is greater than or equal to 1.0 cm, the culturing of microalgae from being in an unfavorable state caused by water evaporating while the biofilm is formed on a liquid surface is sufficiently avoided. When the water depth is less than or equal to 10 m, the handling of a suspended solution containing a medium or microalgae being too difficult can be avoided and it is possible to suppress a decrease in usage efficiency of light for absorbing light in the liquid. When the water depth is 5.0 cm to 30 cm, a lower amount of water in the medium evaporates while the biofilm is formed, the handling of the suspended solution containing a medium or microalgae becomes easy, and the usage efficiency of light improves since the absorption of light due to microalgae infinitesimally existing in water or a medium is minimized. In addition, it is easy to supply carbon dioxide and to release oxygen generated through photosynthesis to the air as the water depth becomes shallower.

In the present invention, a medium mainly plays a role in supplying nutrients for culturing. Therefore, if there are nutrient components in liquid, it is possible to make the depth of a liquid medium shallow, and to thereby greatly reduce the amount of liquid in the medium. Accordingly, the handling of movement of liquid becomes easy as well as the cost for water or the medium being greatly reduced. In addition, the treatment cost of the used medium becomes cheap because a smaller amount of liquid medium. Furthermore, the shallow water depth is advantageous also for diffusion of carbon dioxide (CO₂) in a gas phase to the entire liquid medium tank. In addition, the deeper the water depth of a culture pond, the greater the light absorbed by the medium. However, since the depth thereof is shallow, the decrease in the amount of light due to the absorption is minimized.

### [Carbon Dioxide]

In the present invention, it is preferable that the culturing is performed without intentionally using means for supplying carbon dioxide to a medium. That is, it is preferable that a method of supplying gas containing carbon dioxide to a medium through bubbling is not used. This is done to prevent a biofilm (film-like structure or three-dimensional structure) formed of microalgae on a liquid surface from being destroyed due to the bubbling. However, when the destruction is partially caused, carbon dioxide may be supplied through bubbling. In addition, the method of floating microalgae on a liquid surface through bubbling is disclosed in JP-A-2001-340847, JP-A-2007-160178, JP-A-62-213892, JP-A-1-131711, and the like. However, means for supplying carbon dioxide may also be employed in addition to forcibly floating microalgae on a liquid surface through the method. The method of not using the bubbling is extremely effective from the viewpoint that it is unnecessary to install gas piping for supplying carbon dioxide; it is generally difficult to secure land in the vicinity of a supply source of carbon dioxide, for example, a thermal power station or a steelworks; and it is extremely difficult to perform control for uniformly supplying carbon dioxide to a wide area. Moreover, the method of not using the bubbling is extremely effective from the viewpoint that the method results in great reduction in cost since it is possible to directly use carbon dioxide in the gas phase.

In the present invention, it is preferable not to use means for intentionally supplying carbon dioxide to the inside of a medium. However, a case, in which carbon dioxide is supplied to the inside of a medium such that carbon dioxide in the gas phase passes through microalgae on a liquid surface or a region in which microalgae do not exist, is defined as not corresponding to the means. There are many portions in which the liquid surface of a medium is directly brought into contact with gas containing carbon dioxide before a film-like structure formed of microalgae is formed on the liquid surface. In this case, carbon dioxide dissolves in the medium through the liquid surface. However, it is not considered that carbon dioxide is intentionally supplied in this case. In addition, carbon dioxide dissolves in the medium through a film-like structure or a three-dimensional structure even after the film-like structure or the three-dimensional structure is formed on the liquid surface. However, it is not considered that carbon dioxide is intentionally supplied to the medium in this case as well.

In the present invention, it is desirable to use carbon dioxide in the air from the viewpoint that it is advantageous in cost. However, it is also possible to use carbon dioxide having a higher concentration than that in the air. In this case, it is desirable to perform culturing using a closed culture container in order to prevent the loss of carbon dioxide due to diffusion. The concentration of carbon dioxide in the gas phase in this case is not particularly limited as long as it is possible to achieve the effect of the present invention, but is preferably greater than or equal to the concentration of carbon dioxide in the air and less than 20 volume%, more preferably 0.1 volume% to 15 volume%, and particularly preferably 0.1 volume% to 10 volume%.

The concentration of carbon dioxide in the air is generally understood to be about 0.04 volume%.

That is, the present invention is advantageous since it is possible to use carbon dioxide in the air phase. This is because it is easy to take up carbon dioxide in the air phase since microalgae for which a large amount of growth is desirable are on the liquid surface. In this case, piping for carbon dioxide or bubbling of carbon dioxide is minimized, and therefore, it is possible to reduce the cost of producing algal bodies.

### [Other Culture Conditions]

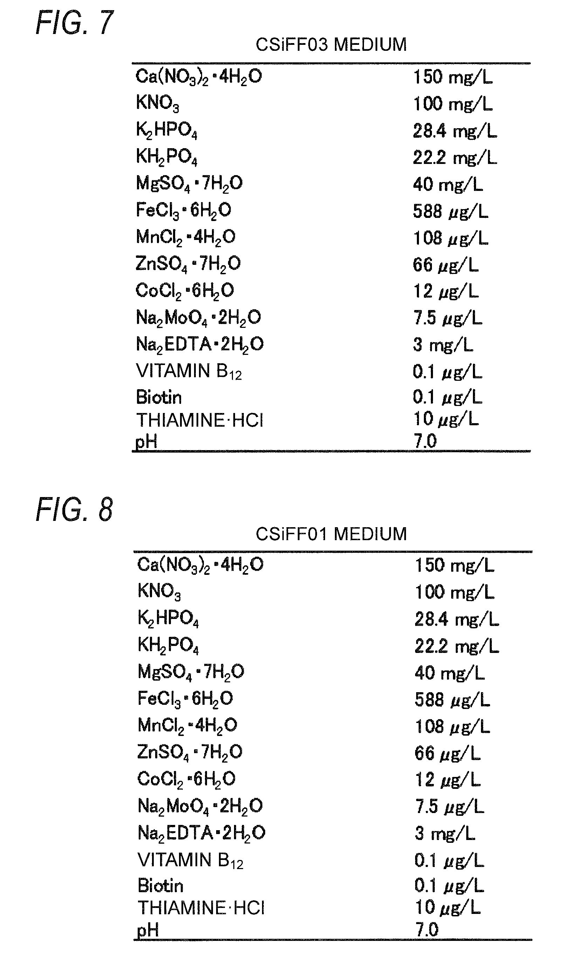

The pH of a liquid medium (culture solution) immediately after starting the culturing is preferably within a range of 2 to 11, more preferably within a range of 5 to 9, and particularly preferably within a range of 5 to 7. This is because it is possible to favorably increase the proliferation rate of microalgae. In addition, in the medium such as a CSiFF03 (of which the composition is shown in Fig. 7) medium which is high in pH and easily generates deposits, generation of deposits is suppressed at a weakly acidic pH. In addition, it is desirable to start the culturing under the weakly acidic conditions from the viewpoint that the number of algae increases on the liquid surface compared to algae on the bottom surface. In addition, it is preferable to select the pH depending on the kinds of microalgae from the viewpoint that a favorable pH changes depending on the kinds of microalgae. In some cases, the pH immediately after starting the culturing and the pH after starting the culturing changes in accordance with the proliferation of microalgae. Therefore, the pH of the liquid medium immediately after starting the culturing may be different from the pH at the time of collecting the microalgae.

The culture temperature can be selected in accordance with the kinds of microalgae, but is preferably 0°C to 90°C, more preferably 15°C to 50°C, and particularly preferably higher than or equal to 20°C and less than 40°C. When the culture temperature is higher than or equal to 20°C and less than 40°C, the proliferation rate of microalgae is sufficiently high, and particularly in the case of AVFF007 strains, the proliferation rate is particularly highest when the culturing is performed at 37°C.

The lower limit initial alga body concentration of microalgae is not particularly limited since the microalgae can proliferate as time passes as long as there is one algal body in the culture solution, but is preferably higher than or equal to 1 cell/mL, more preferably higher than or equal to 1000 cells/mL, and still more preferably higher than or equal to 1 x 10⁴ cells/mL. The upper limit initial alga body concentration of microalgae is not particularly limited since the microalgae can proliferate at any high concentration, but is preferably lower than or equal to 10000 x 10⁴ cells/mL, more preferably lower than or equal to 1000 x 10⁴ cells/mL, and still more preferably lower than or equal to 500 x 10⁴ cells/mL from the viewpoint that when the concentration is higher than a certain concentration, the ratio of the number of algal bodies after the proliferation to the number of input algal bodies decreases as the concentration of the algal bodies becomes higher.

In the present invention, it is possible to mix a medium, which has been used for culturing once, with a medium which is newly prepared. By doing this, it is possible to reduce the amount of water used while there is a case where the growth quantity of microalgae decreases. In addition, a method of adding a high-concentration medium can be considered as a method for suppressing the decrease in the growth quantity of microalgae, and it is possible to use these methods in the present invention.

In the present invention, the pre-culturing period in the case of performing the liquid surface-floating culture is preferably 1 day to 300 days, more preferably 3 days to 100 days, and still more preferably 7 days to 50 days.

In regard to the period of liquid surface-floating culture, it is possible to continue the culturing as long as the microalgae grow, but in general, the period thereof is preferably 1 day to 100 days, more preferably 7 days to 50 days, and still more preferably 10 days to 30 days. For example, the period of liquid surface-floating culture includes a time period from (f) to (g), from (g) to (a), from (j) to (k), or the like in Fig. 1.

In the present invention, it is possible to add a substance, which has a buffer action, to a medium for maintaining a constant pH in the medium. In general, it is known that microalgae release various substances outside the bacterial cell in accordance with survival or proliferation. Therefore, it can be considered that the pH in the medium may change depending on the released substances and the environment may change to an environment in which the culturing of microalgae is not favorably performed. It is preferable to add the substance having a buffer action in order to avoid such a phenomenon. Furthermore, microalgae proliferate using carbon dioxide as a carbon source. Therefore, it can also be considered that the pH of a medium decreases in accordance with dissolution of carbon dioxide into the medium and the environment changes to an environment in which the culturing of microalgae is not favorably performed. It is preferable to add the substance having a buffer action in order to avoid this phenomenon also. A well known substance can be used as the substance having a buffer action and there is no restriction on its use. However, 4-(2-hydroxyethyl)-1-piperazineethanesulfonic acid (HEPES) buffer solution, a sodium phosphate buffer solution, a potassium phosphate buffer solution, or the like may be favorably used. The concentrations or the kinds of buffer substances can be determined in accordance with the culture environments of the microalgae.

### [Culture Container]

As the shape of a culture container (culture pond) that can be used in the present invention, a well-known culture container having any form can be used as long as it is possible to maintain the suspended solution of microalgae. For example, it is possible to use a culture container having a cylindrical shape, a rectangular shape, a spherical shape, a plate shape, a tubular shape, and an irregular shape such as that of a plastic bag. In addition, as the culture container that can be used in the present invention, various well-known methods using types such as an open pond type, a raceway type, and a tube type (J. Biotechnol., 92, 113, 2001) can be used. Examples of the forms that can be used as the culture container include culture containers disclosed in Journal of Biotechnology 70 (1999) 313-321, Eng. Life Sci. 9, 165-177 (2009). Among these, use of the open pond type or the raceway type is preferable in view of cost.

As the culture container that can be used in the present invention, either an open type or a closed type can be used. However, a closed type culture container is favorably used in order to prevent microorganisms other than the microorganisms for culturing, or debris from being mixed in; to suppress evaporation of a medium, to prevent a biofilm (structure of microalgae) on a liquid surface from being destroyed or moved due to wind; and to prevent carbon dioxide from diffusing outside the culture container when using carbon dioxide having a concentration greater than or equal to the concentration of carbon dioxide in the air.

### [Light Source and Amount of Light]

As light sources that can be used for the light irradiation, any light source can be used. However, it is possible to use sunlight, LED light, a fluorescent lamp, an incandescent lamp, xenon lamp light, a halogen lamp, and the like. Among these, it is preferable to use sunlight as natural energy, an LED having a good luminous efficiency, or a fluorescent lamp that can be simply used.

The amount of light is preferably 100 lux to 1000000 lux and more preferably 300 lux to 500000 lux. The most preferable amount of light is 1000 lux to 200000 lux. A larger amount of light is preferable in view of improvement of the proliferation rate of microalgae. If the amount of light is greater than or equal to 1000 lux, the growth rate of microalgae is sufficiently high. If the amount of light is less than or equal to 200000 lux, generation of light damage can be suppressed and the proportion, at which the proliferation rate of microalgae is decreased or microalgae die, can be sufficiently suppressed.

Light may be radiated through any method such as continuous irradiation, and repetition of irradiation and non-irradiation at a constant time interval, but it is preferable that light be turned on and off at a time interval of 12 hours since this is close to a natural state. From results of tests, for the liquid surface-floating culture, it is preferable that continuous irradiation is performed immediately after starting of the culturing and that irradiation is performed by turning light on and off at a time interval of 12 hours from a middle stage of the culturing to a later stage of the culturing.

The wavelength of light is not particularly limited, and any wavelength can be used as long as the wavelength is a wavelength at which photosynthesis can be performed. A preferable wavelength is a wavelength of sunlight or a wavelength similar to that of sunlight. However, an example in which the growth rate of photosynthetic organisms is improved by radiating a single wavelength has been reported, and in the present invention, it is preferable to use such an irradiation method.

Meanwhile, from the viewpoint of cost, irradiation using light without controlling the wavelength is more financially advantageous compared to irradiation using light having a single wavelength. Therefore, use of sunlight is most advantageous in view of cost.

### [Number of Times of Repetition of Culturing]

The number of times of repetition of culturing referred to in the present invention is the number of times of repetition of a process of culturing microalgae and a process of collecting the microalgae. That is, the number of times of repetition of culturing refers to the number of times of repetition of a process from (a) to (g), and further returning to (a), through (b), (d), or (f), in Fig. 1. In the present invention, even if a film-like structure formed of microalgae on a liquid surface is collected through transfer, microalgae remain on a bottom surface, a few microalgae remain on the liquid surface even after the collection, or microalgae, are intentionally made remain on the liquid surface. Therefore, it is possible to repeat the above-described process as many time as required, as long as there are still nutrient components required for proliferation of the microalgae. The number of times of culturing can be determined depending on the amount of nutrient components for proliferation of microalgae, the existence state of microorganisms other than target microorganisms, or the like. Accordingly, the number of culture containers can be reduced and a user can be released from troublesome caused by movement of a culture solution containing microalgae.

### [Biofilm Formed on Liquid Surface]

The present invention also relates to a biofilm formed on a liquid surface through the method of culturing microalgae of the present invention.

The biofilm is generally a film-like structure and refers to a state in which microalgae are connected to each other to form the film-like structure. In order to connect the microalgae to each other, it is necessary to bind the microalgae to each other through, for example, a chemical action of substances (for example, polysaccharides) such as an intracellular matrix, by releasing the substances from the microalgae. That is, the film-like structure is in a state in which microalgae are connected to each other to the extent that they are not separated from each other with the movement of a weak water current. In general, in many cases, such a film-like structure is described as a biofilm.

The biofilm according to the present invention may be a uniform film-like structure of aggregates of microalgae without any gaps over the entire culture container, or may be a steric three-dimensional structure in which a portion of such a film-like structure is formed in a shape of a rising bubble. The phenomenon that a portion of the film-like structure rises in the bubble shape is observed in accordance with the progress of proliferation of microalgae. There may be a large number of the structures in the culture container and the sizes of the structures may vary.

Such a three-dimensional structure has a characteristic in that the number of microalgae is increased in a location close to a light source. It is presumed that this is because a surface which comes into contact with an atmospheric layer rather than being within the three-dimensional structure, that is, a surface close to a light source includes plenty of carbon dioxide and light.

The location at which microalgae exist is away from a liquid surface and moves closer to the light source as the three-dimensional structure develops. This reduces the supply of water from the liquid surface and diffusion of heat generated by light irradiation becomes difficult. As a result, the water content ratio is more decreased as the microalgae exist in a location closer to the light source. The decrease in the water content ratio enables simplification of a dehydration process when performing the oil extraction process after the collecting process, and therefore, this is advantageous with respect to cost reduction in production of biomass using microalgae. In general, a treatment of reducing the water content ratio of microalgae is performed using a centrifugal separator during the collecting process. However, in the collecting method using the culturing method of the present invention, it is possible to obtain a water content ratio lower than that of microalgae obtained by a centrifugal separator.

In addition, in some cases, a wrinkle-like structure appears in the film-like structure in accordance with the progress of proliferation of microalgae. The film-like structure may have such a structure.

Furthermore, in some cases, the film-like structure or the steric three-dimensional structure forms a pleated structure or a curtain-like structure in the medium in accordance with the progress of proliferation of microalgae. The film-like structure and the steric three-dimensional structure may have such a structure.

As described above, the film-like structure or the steric three-dimensional structure may have the wrinkle-like structure, the pleated structure, and the curtain-like structure, or the film-like structure may be made to have a steric three-dimensional structure formed with the bubble-like structure. Employing such structures is preferable in view of increasing the quantity of algal bodies per unit area.

It is preferable that the biofilm has an area to the extent that a piece of the biofilm existing on the liquid surface does not escape from a substrate through the liquid surface while performing collection using the substrate. It is more preferable that there is no gap in the biofilm over the entire culture container. For example, such an area can be larger than or equal to 1 cm² and preferably larger than or equal to 10 cm². The area of the biofilm is particularly preferably larger than or equal to 100 cm² and smaller than or equal to the surface area of the liquid surface. The upper limit of such an area is not particularly limited as long as the area is smaller than or equal to the area of the liquid surface of the culture container.

The thickness of a film-like structure is generally within a range of 1 µm to 10000 µm, preferably within a range of 1 µm to 1000 µm, and more preferably 10 µm to 1000 µm.

In a case in which the biofilm according to the present invention is a steric three-dimensional structure formed in a shape of a rising bubble in a portion or a plurality of portions of film-like structure, the height of the steric three-dimensional structure having a liquid surface of a medium as a reference is generally within a range of 0.01 mm to 100 mm, preferably within a range of 0.1 mm to 20 mm, and more preferably within a range of 5 mm to 20 mm.

The weight of dry alga bodies of the biofilm according to the present invention per unit area is preferably greater than or equal to 0.001 mg/cm², more preferably greater than or equal to 0.1 mg/cm², and particularly preferably greater than or equal to 1 mg/cm². The most preferable weight of the dry alga bodies is greater than or equal to 5 mg/cm². This is because it is expected that if the weight of the dry alga bodies per unit area is great, the collected amount of biomass such as oil will be greater. In general, the weight of the dry alga bodies of the biofilm per unit area is less than or equal to 100 mg/cm².

In addition, the film density of the microalgae in the biofilm according to the present invention per unit area is preferably greater than or equal to 100000 cells/cm², more preferably greater than or equal to 1000000 cells/cm², and particularly preferably greater than or equal to 10000000 cells/cm². When the film density of the microalgae in the biofilm per unit area is less than 100000 cells/cm², the formation of the biofilm on the liquid surface cannot be confirmed and the recovery properties of microalgae deteriorate. In contrast, when the film density of the microalgae in the biofilm per unit area is greater than or equal to 10000000 cells/cm², due to the high film density, a strong biofilm is formed on the liquid surface and the recovery properties improve. The greater the film density, the more preferable, and therefore, the upper limit of the film density of the microalgae in the biofilm per unit area is not particularly limited. In general, the film density is less than or equal to 10 billion cells/cm².

It is preferable that the biofilm according to the present invention has a low water content ratio in view of saving labor in a process of removing water and in view of cost reduction. Specifically, the water content ratio of the biofilm is preferably less than or equal to 95 mass%, more preferably less than or equal to 90 mass%, particularly preferably less than or equal to 80 mass%, and most preferably less than or equal to 70 mass%. In general, the water content ratio of the biofilm is greater than or equal to 50 mass%.

The water content ratio is obtained by dividing a value, which is obtained by subtracting the weight of algal bodies after drying from the weight of the algal bodies before drying, by the weight of the algal bodies before the drying, and then by multiplying by 100.

In addition, it is preferable that the biofilm according to the present invention has a high oil content in view of usefulness as biomass. Specifically, the oil content per dry alga body of the biofilm is preferably higher than or equal to 5 mass%, more preferably higher than or equal to 10 mass%, and particularly preferably higher than or equal to 15 mass%. In general, the oil content per dry alga body of the biofilm is lower than or equal to 80 mass%.

Microalgae capable of forming a biofilm, which has the structure, the area in the above-described range, the thickness, the weight of the algal bodies per unit area, the water content ratio, and the oil content which are described above, on a liquid surface are preferable as the microalgae of the present invention for the same reasons as described above.

### [Collecting Process]

The collecting process is a process of peeling off a biofilm (film-like structure or three-dimensional structure) formed of microalgae on a liquid surface from a first substrate after the biofilm is transferred to the first substrate, as shown in, for example, (b) to (c) or (1) to (n) of Fig. 1, or a process of collecting a biofilm (film-like structure or three-dimensional structure) formed of microalgae on a liquid surface using a second substrate as shown in, for example, (d) to (e) or (m) to (o) of Fig. 1.

The present invention also relates to a method of collecting biofilm in which the biofilm formed on a liquid surface is collected by transferring the biofilm to a first substrate.

In addition, the present invention also relates to a method of collecting biofilm in which the biofilm formed on a liquid surface is collected by depositing the biofilm on a second substrate.

Any well-known method can be used for attaching/detaching (collecting) the biofilm from the first substrate as long as the method can peel off the biofilm from the first substrate. For example, a method of peeling off the biofilm from the substrate using a cell scraper or the like, a method of using a stream of water, and a method of using ultrasonic waves can be used. Among these, the method of using a solid material such as a cell scraper which is made of a material that may not damage the surface of the first substrate greatly is preferable. This is because other methods are inefficient since, in some cases, the biofilm is diluted by a medium or the like and it is necessary to re-concentrate the biofilm.

Any well-known method can be used for attaching/detaching (collecting) the biofilm from the second substrate as long as the method can peel off the biofilm from the second substrate. For example, a method using gravity, a method of peeling off the biofilm from the substrate using a solid material such as a cell scraper which is made of a material that may not damage the surface of the first substrate greatly, a method of using a stream of water, and a method of using ultrasonic waves can be used. Among these, the method of using a free fall due to gravity or the method of using the solid material such as the cell scraper is preferable. This is because other methods are inefficient since, in some cases, the biofilm is diluted by a medium or the like and it is necessary to re-concentrate the biofilm. In addition, it is possible to attach/detach algae remaining on the second substrate using the cell scraper or the like after collecting the biofilm using the free fall due to gravity. In the present invention, as described above, collection for performing subsequent culturing and collection for performing a process of extracting biomass are included in the collecting process.

### [Method for Partially Leaving Microalgae]

The method is a method of partially leaving microalgae on a liquid surface in a process of transferring a biofilm on a liquid surface using a first substrate, that is, the process from (b) to (c) of Fig. 1 or the process from (l) to (n) of Fig. 1 for example; and a process of collecting a biofilm on a liquid surface using a second substrate, that is, the process from (d) to (e) of Fig. 1 or the process from (m) to (o) of Fig. 1 for example. In some cases, a firm-like structure can be quickly formed on a liquid surface by partially leaving microalgae on the liquid surface compared to a case where algae are supplied from microalgae existing on a bottom surface or in liquid. Any pattern may be employed as a pattern in which microalgae remain on the liquid surface, and examples thereof include the pattern shown in the process from (a) to (e) of Fig. 2 or a grid pattern through transferring.

In the method of culturing microalgae of the present invention, when collecting a part of a biofilm as a film-like structure or a three-dimensional structure which is formed on the entire surface on a liquid surface in culturing in a first stage, the area of the biofilm collected is preferably 0.1% to 99.9% of the area of the liquid surface, more preferably 20% to 80% thereof, still more preferably 25% to 75% thereof, and most preferably 30% to 70% thereof (that is, the area of a biofilm remaining on the liquid surface is preferably 0.1% to 99.9% of the surface area of the liquid surface, more preferably 20% to 80% thereof, still more preferably 25% to 75% thereof, and most preferably 30% to 70% thereof).

In addition, any of the biofilm supplied on the liquid surface or the biofilm remaining on the liquid surface may be stirred to more subdivide the structure and at least a part of the biofilm may be made to float on the liquid surface. This is because, in some cases, it is possible to increase the proliferation rate by doing this.

### [Transfer of Biofilm]

The transfer referred to in the present invention is a kind of adhesion and is substantially adhesion without proliferation. In the present invention, the transfer refers to an operation of substantially transferring a biofilm formed on a liquid surface to the surface of a first substrate as it is using a first substrate.

As shown in (b) or (I) of Fig. 1, the transfer of a biofilm is a process of transferring a biofilm formed on a liquid surface to the surface of a substrate using a first substrate. In the drawing, the collecting process is performed in a state in which the biofilm is formed on the entire surface in the culture container. In the present invention, the collection may be performed in such a state. Alternately, in the present invention, a transferring process can also be performed in a state in which the biofilm formed of microalgae partially do not exist. In addition, when performing culturing through the method of the present invention, in some cases, the biofilm formed on the liquid surface forms a wrinkle-like shape or is folded and piled, or a pleated film-like structure formed of microalgae grows like an aurora (curtain shape) in liquid. In the present invention, it is possible to perform the transfer even in such a state, and a transferring method and a culturing method using such a method can be included in the present invention.

### [Division Treatment]

The division treatment referred to in the present invention is an operation in which a structure such as a film-like structure or a three-dimensional structure formed of microalgae is partially destroyed and is widely distributed on a liquid surface.

The division can be performed either or both before collection of microalgae or/and after collection of microalgae. However, the division, is preferably performed before collection of microalgae in terms of effectiveness of promoting formation of a three-dimensional structure. Through the formation of a three-dimensional structure, the number of microalgae per unit area is increased and the water content ratio after collection is decreased, which is advantageous to reduce cost in producing biomass of microalgae.

When the division is performed after collection of microalgae, it is possible to allow microalgae exist in a region where there are no microalgae generated through a collecting operation, by performing the dividing operation on such a region, and it is possible to effectively utilize a liquid surface of a culture container. In addition, as described above, through the division treatment, the number of microalgae per unit area is increased and the water content ratio after collection is decreased, which is advantageous to reduce cost in producing biomass of microalgae.

Any method can be used as the dividing method as long as the method can uniformly divide a structure as much as possible in a state where a biofilm on a liquid surface is made to float on the liquid surface as much as possible, and examples thereof include a method of utilizing wind, a method of utilizing a water flow, a method of utilizing vibration such as a wave on a liquid surface, and a method of utilizing a solid material. The method of utilizing a solid material refers to a treatment in which a biofilm on a liquid surface is split using, for example, a tool such as a rod or a screw. In addition, the collecting process can be used in the division treatment as it is. That is, this method uses a phenomenon in which a biofilm remaining on a liquid surface is naturally divided through an operation during collection using a first substrate or a second substrate.

In regard to the degree of division, the thinner the biofilm, the more preferable. However, a stronger operation is required for making the biofilm thinner, and therefore, there is a high possibility that microalgae may sink to the bottom surface. Thus, in the present invention, the biofilm may be made to have a size at which the biofilm can be visibly recognized. The size of the biofilm after the division is preferably 0.01 cm² to 10000 cm², more preferably 0.1 cm² to 1000 cm², and most preferably 0.2 cm² to 100 cm². The division treatment may be performed any number of times during culturing.

In the method of culturing microalgae of the present invention, when performing the division treatment, it is preferable that the division treatment is performed on a liquid surface where a region, in which a biofilm substantially exists, and a region, in which a biofilm does not substantially exist, coexist.

It is more preferable that a divided biofilm through the division treatment is moved to the region in which a biofilm does not substantially exist.

### [Substrate]

The substrate referred to in the present invention is a substrate which is used in (b) or (1) of Fig. 1 or (d) or (m) of Fig. 1 and is used for transferring or collecting a biofilm on a liquid surface.

It is preferable that the area of a first substrate and a second substrate is smaller than that of a liquid surface of a culture solution in a culture container.

The substrate according to the present invention is preferably a substrate which has a plurality of sites for collecting a biofilm. That is, in the method of culturing microalgae of the present invention, it is preferable that a biofilm formed on a liquid surface in the above-described culturing in the first stage is collected using one sheet of a substrate which has the plurality of sites for collecting a biofilm. This is because it is easy to transfer the biofilm obtained through the culturing in the first stage into a plurality of second culture containers.

### [Material]

The materials of the culture container, the first substrate, and the second substrate that can be used in the present invention are not particularly limited, and well-known materials can be used. For example, it is possible to use a material formed of an organic polymer compound, an inorganic compound, or a composite thereof. In addition, it is also possible to use a mixture thereof.

Polyethylene derivatives, polyvinyl chloride derivatives, polyester derivatives, polyamide derivatives, polystyrene derivatives, polypropylene derivatives, polyacrylic derivatives, polyethylene terephthalate derivatives, polybutylene terephthalate derivatives, nylon derivatives, polyethylene naphthalate derivatives, polycarbonate derivatives, polyvinylidene chloride derivatives, polyacrylonitrile derivatives, polyvinyl alcohol derivatives, polyethersulfone derivatives, polyarylate derivatives, allyl diglycol carbonate derivatives, ethylene-vinyl acetate copolymer derivatives, fluorine resin derivatives, polylactic acid derivatives, acrylic resin derivatives, ethylene-vinyl alcohol copolymers, ethylene-methacrylic acid copolymers, and the like can be used as the organic polymer compound.

Glass, ceramics, concrete, and the like can be used as the inorganic compound.

Alloys such as iron, aluminum, copper, or stainless steel can be used as a metallic compound.

Among these, it is preferable that a portion of the materials of the first substrate, the second substrate, or the culture container is formed of at least one selected from glass, polyethylene, polypropylene, nylon, polystyrene, vinyl chloride, and polyester.

In addition, the materials of the culture container, the first substrate, and the second substrate may be the same as each other or different from each other.

In addition, when using a closed type culture container, the light receiving surface may be made of a material through which light is transmitted, and a transparent material is more preferable.

### [First Substrate]

As the first substrate, a substrate which covers the entire surface of a culture container is used in the drawing. However, the substrate which covers the entirety of the culture container may be used in this manner or a first substrate which can cover only a part of the culture container may be used. Transfer of microalgae on a liquid surface using the first substrate may be performed plural times. This is because the transfer rate is improved by performing the transfer plural times. In addition, the first substrate may be reused many times.

### [Second Substrate]

As the second substrate, a substrate which has a surface area smaller than that of a culture container is preferably used. In addition, the collection may be performed plural times. This is because the collection rate is improved by performing the collection plural times. Only one sheet of a second substrate is used in the drawing, but a plurality of second substrates can be simultaneously used. Accordingly, the collection rate is improved. In addition, the second substrate may be reused many times.

### [Film-like Structure]

The film-like structure referred to in the present invention is a structure which is formed of microalgae that are aggregated with each other through an intercellular substance. In the present invention, the film-like structure floating on a liquid surface is a significant characteristic. In general, the structure changes from the film-like structure to a three-dimensional structure along with proliferation, and is mainly a two-dimensional structure.

### [Three-dimensional Structure]

The three-dimensional structure referred to in the present invention is a structure which is formed of microalgae that are aggregated with each other through an intercellular substance. In the present invention, the three-dimensional structure floating on a liquid surface is a significant characteristic. In general, the structure changes from the film-like structure to the three-dimensional structure along with proliferation, and there is also a situation where the film-like structure and the three-dimensional structure coexist.

A structure in which a film-like structure forms a wrinkle-like shape, a structure in which a film-like structure extends in a pleated shape or a curtain shape under a liquid surface, and the like are included in the three-dimensional structure. The three-dimensional structure refers to a structure in which at least one bubble is formed on the liquid surface or several bubbles are piled on the liquid surface.

### [Biomass and Oil]

The present invention also relates to biomass and oil obtained from a biofilm formed on a liquid surface through the method of culturing microalgae of the present invention.

In addition, the present invention also relates to a method of producing biomass derived from microalgae, the method including the method of culturing microalgae of the present invention.

In the present invention, "biomass" refers to a renewable organic resource derived from organisms excluding a fossil resource, and examples thereof include substances, food products, materials, fuel, and resources derived from organisms.

In the present invention, "oil" refers to a flammable fluid substance, is a compound mainly formed of carbon and hydrogen, and is a substance occasionally containing oxygen, nitrogen, and the like. In general, the oil is a mixed material and is a substance which is extracted using a low-polarity solvent such as hexane or acetone. The composition thereof is formed of hydrocarbon compounds, fatty acids, triglycerides, or the like. In addition, the composition thereof is esterified to be used as biodiesel.

The method of collecting biomass and oil contained in a biofilm according to the present invention is not particularly limited as long as the method does not impair the effect of the present invention.

As a general collecting method of oil as an example of the biomass, after obtaining dry alga bodies by heat-drying a biofilm, cell-disruption is performed as necessary to extract oil using an organic solvent. In general, the extracted oil contains impurities such as chlorophyll, and therefore, it is necessary to perform purification. There is a case of performing purification through silica gel column chromatography or performing purification through distillation (for example, a distillation method disclosed in JP-T-2010-539300).

In addition, there is a method of extracting oil in algal bodies using an organic solvent after crushing microalgae through an ultrasonic treatment or crushing microalgae using protease or an enzyme after a solution of high-concentration microalgae is prepared (for example, a method disclosed in JP-T-2010-530741).

In this manner, the biofilm according to the present invention is useful as biomass fuel. That is, the present invention also relates to a method of producing biomass fuel in which the biofilm collected through the method of collecting the biofilm according to the present invention is used as fuel.

### [Dry Alga Bodies]

The dry alga bodies in the present invention are obtained by drying the biofilm according to the present invention.

The method of drying the biofilm is not particularly limited as long as the method can remove water from the biofilm. Examples thereof include a method of sun-drying a biofilm; a method of blowing dry air onto a biofilm; a method of freeze-drying a biofilm; and a method of heating a biofilm. Among these, the dry method through the freeze-drying is preferable in view of being capable of suppressing decomposition of components contained in the biofilm and the heat-drying method is preferable in view of being capable of efficiently perform the drying in a short period of time.

### Examples

The present invention will be further described in detail with reference to the following Examples, but the present invention is not limited to the following Examples.

### [Example 1]

A test sample was prepared by collecting a biofilm as a film-like structure which was formed by culturing (pre-culturing) through liquid surface-floating culture. In the pre-culturing, AVFF007 strains were used as microalgae and were used by adjusting the initial alga body concentration to 10 x 10⁴ cells/mL. As the culture conditions, the culturing was performed using a plant bioshelf for tissue culture (AV152261-12-2, Ikeda Scientific Co., Ltd.) and by adding 8 mL of a suspended solution of the above-described microalgae to a 6 hole plate (1-835501, plate for culturing microorganisms, As One Corporation, diameter of 3.5 cm). Light irradiation was performed by turning on and off a fluorescent lamp at 4000 lux every 12 hours; a CSiFF01 medium which has the composition shown in Fig. 8 was used as a medium in an amount which corresponds to 8 mm of water depth; and stationary culture was performed for 20 days at room temperature (23°C) in the above-described 6 hole plate. The collection of the film-like structure formed on the liquid surface was performed through collection through transfer using a polyethylene film.

The collected substance of microalgae (film-like structure) was suspended in a 3 mL of a CSiFF01 medium in wells in a 6 hole plate, into which the medium was previously put, by being allowed to sink in the medium in a manner that a transferred surface of the microalgae was set as an upper surface and by peeling off the microalgae on the polyethylene film using a cell separator (MS-93100, Sumitomo Bakelite Co., Ltd.). After putting a total amount of the suspended solution into the 5 mL tube for homogenizing (TM-655S, Tomy Seiko Co., Ltd.), the tube was set in a beads cell disrupter (MS-100, Tomy Seiko Co., Ltd.), and then, a homogenization treatment lasting for 20 seconds was performed 3 times at 4200 rpm without using beads for the cell disrupter. The concentration of algal bodies in the suspended solution was calculated from turbidity (absorbance at 660 nm). A calculation expression was calculated by calculating the number of algal bodies from the turbidity after obtaining a relational expression between the turbidity and the number of algal bodies in advance. In the case of the AVFF007 strains, an expression of y = 115.26x² + 782x + 25.63 was satisfied. y is the number of algal bodies [unit: x 10⁴ cells/mL] and x is the absorbance at 660 nm.

Culturing (main culturing) in a first stage was started using the microalgae obtained from the pre-culturing. 60 mL of a dispersed solution at an initial use alga body concentration of 20 x 10⁴ cells/mL was prepared by diluting the suspended solution obtained in the pre-culturing with a CSiFF03 medium. This solution was added to 16 styrene rectangular type 1 cases (inner dimension of 3.1 cm x 3.1 cm, As One Corporation) by 3.85 mL (water depth of 4 mm) which were then put into a vacuum desiccator (1-070-01, As One Corporation) without being covered with lids. After setting a 5% carbon dioxide atmosphere, stationary culture was performed for 7 days at room temperature (23°C) by performing light irradiation in which turning on and off of a fluorescent lamp at 4000 lux was shifted every 12 hours using a plant bioshelf for tissue culture.

After confirming that a film-like structure has been formed on the liquid surface in the culturing in the first stage, a polyethylene film having a length the same as the inner dimension of the styrene rectangular type 1 case was inserted into the liquid along the wall surface of the rectangular case. Then, the film was slid in a horizontal direction and the film-like structure formed on the liquid surface was collected by being deposited thereon. The quantity of dry alga bodies per unit area which was obtained in the culturing in the first stage and was calculated through a heating treatment at 110°C became 0.021 mg/cm².

Next, culturing (main culturing) in a second stage was started after performing the following procedure (division treatment or the like). 90 mL (water depth of 8 mm) of a CSiFF03 medium was put into a styrene rectangular type 8 case (inner dimension of 7.85 cm x 14.25 cm, As One Corporation), and then, the collected substance of algal bodies formed in the culturing in the first stage was put therein such that the collected substance floated on a liquid surface in the vicinity of the center of the above-described container. Since the collected substance of algal bodies was localized to the liquid surface, division treatment was performed so as to make the microalgae on the liquid surface be uniformly distributed on the entire surface of the culture container. The majority of the microalgae floated on the liquid surface, but some of the microalgae sunk to the bottom surface of the culture container. 8 containers in which microalgae are made to float on each liquid surface and which were prepared in this manner were prepared; and four styrene rectangular type 8 cases were put into each of two vacuum desiccators in a state where the styrene rectangular type 8 cases were not covered with lids, to start the culturing in the second stage using a plant bioshelf for tissue culture. The culture conditions were the same as those in the culturing in the first stage. In addition, carbon dioxide was prepared and supplied every evaluation day.

On day 2 of the culturing in the second stage, collection was attempted through the same method as the collecting method during the culturing in the first stage using a nylon film which was cut in a length corresponding to a short side of the styrene rectangular type 8 case. However, the collected amount was small since a biofilm was not entirely formed on the liquid surface of the culture container and microalgae escaped through a gap between the nylon film and the wall surface of the culture container. For this reason, collection was performed through a transferring method using a polyethylene film, that is, the same method as that of collecting microalgae during the pre-culturing. After day 2 of the culturing, collection was performed through the same method as the collecting method in the culturing in the first stage except for the material used therefor.

On day 10 of the culturing in the second stage, a state was observed in which the biofilm as a film-like structure spread over the entirety of the liquid surface and many bubbles were generated. On day 21 of the culturing in the second stage, a three-dimensional structure was formed on the liquid surface.

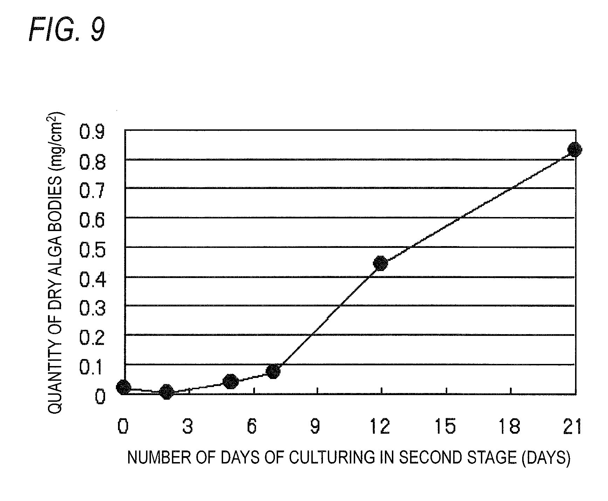

The relationship between the number of days of culturing in the second stage and the quantity of dry algal bodies of the biofilm formed on the liquid surface is shown in Fig. 9.

It is obvious from the above results that it is possible to perform culturing so as to form a biofilm on a liquid surface from a state where microalgae float on the liquid surface. The quantity of dry algal bodies was calculated through a heating treatment at 110°C.

### [Reference Example 1]

Pre-culturing and culturing in a first stage were performed through the same method as that in Example 1.

Microalgae were collected through the same method as that in the culturing in the first stage in Example 1; the collected microalgae were put into a 5 mL tube for homogenizing into which 2 mL of a CSiFF03 medium was previously put; and a homogenization treatment was performed three times for 20 seconds at a shaking rate of 4200 rpm to prepare a dispersed solution of microalgae.

Next, culturing in a second stage was started. 88 mL of a CSiFF03 medium was put into a styrene rectangular type 8 case, and then, the entire amount (2 mL) of the dispersed solution of microalgae derived from the culturing in the first stage was put therein. Accordingly, the total amount of liquid became 90 mL and the water depth became 8 mm. Two styrene rectangular type 8 cases which were prepared in this manner were prepared; the media containing microalgae in the containers was stirred well; and then, the styrene rectangular type 8 cases were put into one vacuum desiccator in a state where the styrene rectangular type 8 cases were not covered with lids, to start the culturing in the second stage using a plant bioshelf for tissue culture. The culture conditions are the same as those in Example 1.

That is, the culturing in the second stage was started from the state of the biofilm floating on the liquid surface, using microalgae as the biofilm formed on the liquid surface in the culturing in the first stage in Example 1. However, in Reference Example 1, the culturing in the second stage was started using dispersed solution of microalgae which was prepared using the biofilm formed on the liquid surface in the culturing in the first stage.

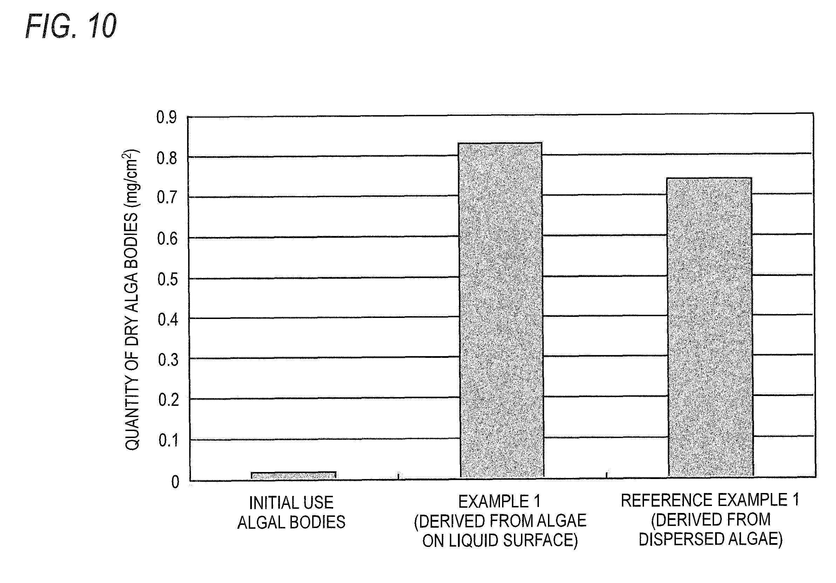

The result of quantity of dry algal bodies of the biofilm formed on the liquid surface in each of Example 1 and Reference Example 1 21 days after the start of the culturing in the second stage and the result (denoted as "day 0 of culturing" in Fig. 10) of quantity of dry algal bodies 0 day after the start of the culturing in the second stage were shown together in Fig. 10.

The result of Example 1 in which the culturing in the second stage was started from the state of the biofilm floating on the liquid surface and the result of Reference Example 1 in which the culturing in the second stage was started from the dispersed state are almost the same as each other (that is, the proliferation rates of microalgae in the culturing in the second stage in Example 1 and Reference Example 1 are almost the same as each other), and the results thereof show that it is possible to perform culturing so as to form a biofilm on a liquid surface from the state of the biofilm floating on the liquid surface.

### [Example 2]

Pre-culturing and collection of microalgae were performed through the same method as that in Example 1.

Culturing in a first stage was performed through the same method as that in Example 1. However, the initial used alga body concentration was changed to 10 x 10⁴ cells/mL, and a styrene rectangular type 8 case was used as a culture container. In addition, a slide glass was used as a substrate for collection.

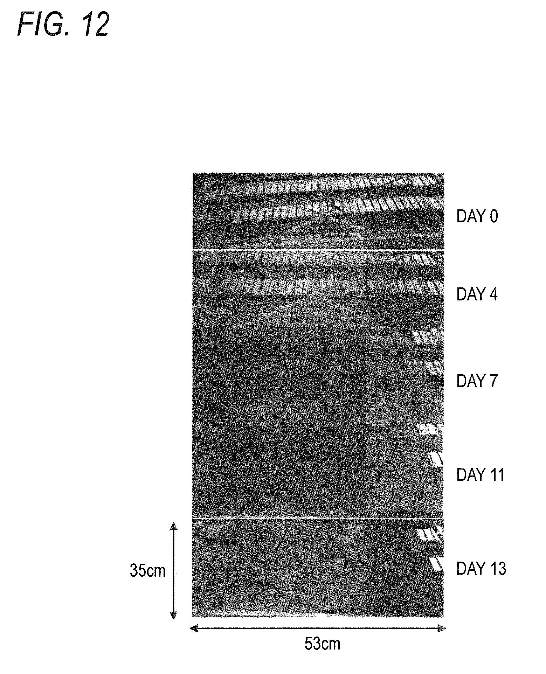

Microalgae collected through the collecting method using a substrate for collection were applied on a liquid surface of a crystal container (inner dimension of 53 cm x 35 cm, 5-379-01, As One Corporation) into which 3 L (water depth of 1.6 cm) of a CSi medium having the composition shown in Fig. 11 was put. In Example 2, a division treatment or the like was not performed before culturing in a second stage was performed unlike in Example 1. Therefore, as shown in the culturing after 0 day in Fig. 12, the culturing in the second stage was started from a state of microalgae staying in one site on the liquid surface.

The crystal container was placed on a plant bioshelf and the culturing in the second stage was started under the same conditions as those in Example 1. The culturing in the second stage was performed in an open system and distilled water was added thereto as necessary due to evaporation of water. In addition, carbon dioxide was supplied only from the air.

As shown at day 7 of culturing in Fig. 12 which shows a state 7 days after the start of the culturing in the second stage, microalgae were proliferated until the microalgae spread over the almost entire surface of the culture container, after one week.

As shown at day 13 of culturing in Fig. 12 which shows a state 13 days after the start of the culturing, the culture container was almost covered with microalgae which proliferated until a folded structure of algae floating on a liquid surface in liquid could be seen. From the above, it was possible to perform liquid surface-floating culture of microalgae even without performing a division treatment.

### [Example 3]

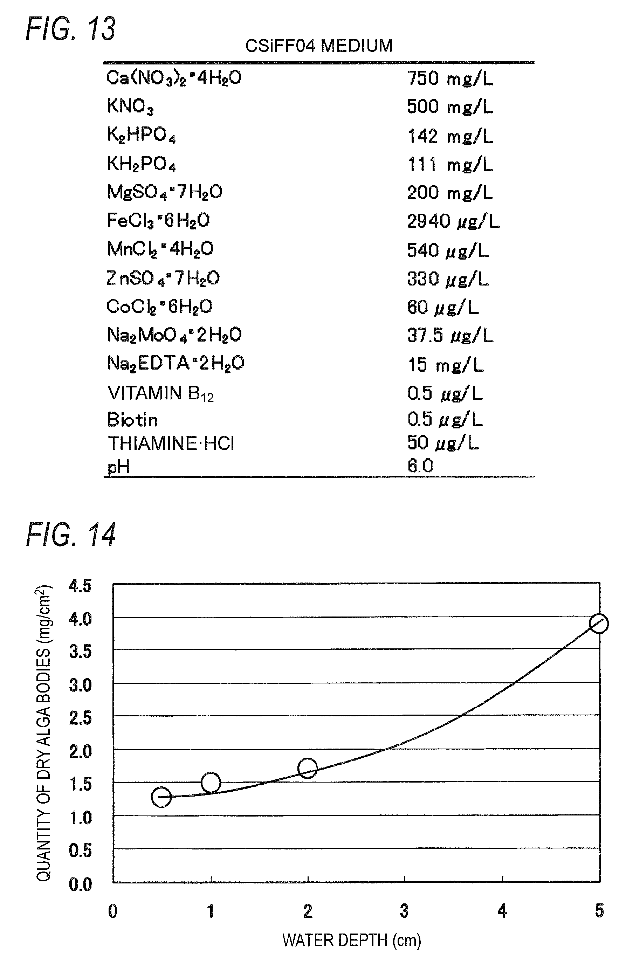

Pre-culturing and collection of microalgae were performed through the same method as that in Example 1. A CSiFF04 medium having the composition shown in Fig. 13 was used as a medium for preparing a dispersed solution of microalgae after the collection. Culturing in a first stage was performed through the same method as that in Example 1. However, the initial use alga body concentration was changed to 50 x 10⁴ cells/mL, and a polystyrene case no. 23 was used as a culture container. Moreover, 20 containers, into each of which 9.4 mL of the dispersed solution of microalgae, that is, the water depth of the containers becoming 1.5 cm was put, were prepared and the amount of light was changed to 15000 lux. In addition, in Example 3, the culturing in the first stage was performed for 6 days and a biofilm formed on a liquid surface was collected using a nylon film instead of the polyethylene film. Similarly to Example 1, the quantity of dry alga bodies per unit area which was obtained in the culturing in the first stage, the quantity of dry alga bodies being calculated through a heating treatment, was 0.123 mg/cm². The biofilm formed on the liquid surface had a structure close to a film-like structure in the method in Example 1, but formed a three-dimensional structure in Example 3.

Culturing in a second stage was performed through the same method as that in Example 1 after performing the following procedure (division treatment or the like). However, the method of introducing the liquid surface-floating biofilm obtained through culturing in the first stage to a liquid surface of a medium in the culturing in the second stage was performed through the method shown in Fig. 3.

Specifically, the polystyrene case No. 23 (corresponding to a culture container 2 in Fig. 3) formed with the biofilm on the liquid surface was immersed in a staining tray (corresponding to a culture container 1 in Fig. 3) into which a medium was put, and at the same time, the biofilm was made to float on a liquid surface of the staining tray from the polystyrene case No. 23. Next, the biofilm floated on the liquid surface of the staining tray was made not to be collected by the polystyrene case No. 23 again, through lifting of the polystyrene case No. 23 above the liquid surface of the staining tray while shifting the polystyrene case No. 23 from the position immersed in the staining tray. The biofilm floated on the staining tray was subjected to a division treatment so as to be uniformly distributed on the liquid surface using a pincette. In cases where the water depth was 0.5 cm and 1 cm, the treatment was performed after making the water depth deep by inclining the staining tray. In addition, as culture containers and the amount of medium, three staining trays (74 mm inner dimension x 94 mm x 63 mm tall, 1-1413-01, As One Corporation) for each water depth were prepared by putting 24.8 mL (water depth of 0.5 cm), 59.6 mL (water depth of 1 cm), 129.1 mL (water depth of 2 cm), and 337.8 mL (water depth of 5 cm) of CSiFF04 media into the staining trays. In addition, light was blocked from the side surfaces of the staining trays using black tape in accordance with the water depth. The period of culturing in the second stage was set to 14 days.

The relationship between the water depth in the culturing in the second stage and the quantity of dry alga bodies of the biofilm formed on the liquid surface after performing the culturing in the second stage for 14 days is shown in Fig. 14.

A biofilm as a three-dimensional structure formed of microalgae on the liquid surface was formed in Example 3, and the amount of proliferating microalgae became larger as the water depth became deeper as shown in Fig. 14.

From the above, it was found that it was also possible to perform culturing so as to form a biofilm on a liquid surface as follows. A biofilm as a three-dimensional structure formed of microalgae on a liquid surface was formed in culturing in a first stage and the total amount of the biofilm was sunk in liquid of a culture container having an area larger than that used in the culturing in a second stage. At the same time, the entire quantity of the microalgae formed in the culturing in the first stage was transferred onto a liquid surface of the culture container used in the culturing in the second stage and the culture container used in the culturing in the first stage was removed from the culture container used in the culturing in the second stage. Then, the microalgae transferred onto the liquid surface of the culture container used in the culturing in the second stage were subjected to a division treatment to perform the culturing (continuous culturing) in the second stage.

It is considered that the proliferation rate of microalgae was more increased as the water depth became deeper due to the increased amount of the medium component in the medium.

### [Example 4]

Pre-culturing and collection of microalgae were performed through the same method as that in Example 1. The period of the pre-culturing in Example 4 was set to 33 days.

Culturing in a first stage was performed through the same method as that in Example 1. However, a CSiFF04 medium was used as a medium and the initial use alga body concentration was changed to 50 x 10⁴ cells/mL, and the amount of light was changed to 4000 lux to prepare 1150 mL of a suspended solution of microalgae. In addition, 5 styrene rectangular type 8 cases, into which each 220 mL of a suspended solution of microalgae was put, were prepared as culture containers, and the culturing in the first stage was started by placing the containers, which were covered with lids, on a plant bioshelf for tissue culture without using a vacuum desiccator. Accordingly, the concentration of carbon dioxide was a concentration in the air remaining in the containers.

After the culturing in the first stage was performed for 7 days, a biofilm on a liquid surface was collected through the same method as the collecting method of a biofilm in the culturing in the first stage in Example 1. However, in Example 4, the collected areas of the biofilm formed on the liquid surface were changed using nylon films of which the sizes were different from each other in order to variously change the collected amount of the biofilm formed on the liquid surface through the culturing in the first stage. The quantity of dry alga bodies of collected substance of the biofilm was calculated through drying by dry heat.

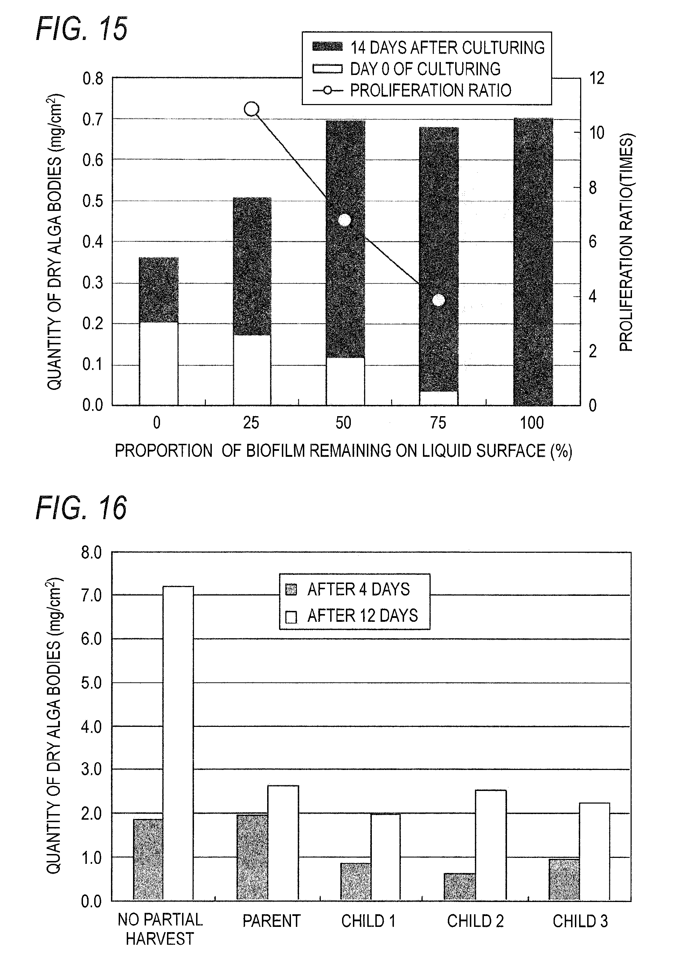

With respect to a biofilm formed on a liquid surface after the culturing in the first stage was performed for 7 days, the quantity of dry alga bodies of the collected substance (that is, the proportion of the biofilm that remained on the liquid surface being 0%) of microalgae which was the biofilm on the entire liquid surface (that is, 100%) based on the area was obtained. Similarly, the quantity of dry alga bodies was obtained in cases where the proportions to be collected were set to 75%, 50%, 25%, and 0% (that is, the proportions of the biofilms that remained on the liquid surface respectively being 25%, 50%, 75%, and 100%) based on the area. The results of the quantities of dry alga bodies were shown by white bars in Fig. 15. That is, the white bar 0 days after the culturing is the quantity of dry alga bodies obtained through collecting the biofilm formed on the liquid surface through the culturing in the first stage. The quantity of algal bodies was largest in a case where the quantity of algal bodies that remained on the liquid surface was 0%, that is, in a case where microalgae on the entire liquid surface were collected, and the quantity of collected algal bodies was decreased in accordance with an increase in the proportion of the biofilm that remained on the liquid surface.

A division treatment of the biofilm was performed by generating a wave on the liquid surface after microalgae as the biofilm on the liquid surface were collected.

After the division treatment, culturing in a second stage was started. The culture conditions were the same as those in the culturing in the first stage. In addition, the medium in the culturing in the first stage was continuously used as it was, as a medium in the culturing in the second stage. The entire quantity of microalgae as the biofilm formed on the liquid surface 14 days after the start of the culturing in the second stage was collected.

With respect to each of the above-described proportions of the biofilms that remained on the liquid surface after the culturing in the first stage, the results of the quantity of dry alga bodies of the microalgae as the biofilm formed on the liquid surface 14 days after the start of the culturing in the second stage were shown by black bars in Fig. 15.

That is, the total value of the white bar and the black bar becomes the total quantity of dry alga bodies of microalgae cultured on the culture container through the culturing in the first stage and the second stage. The quantities of dry alga bodies of microalgae which were harvested through the culturing in the first stage and the culturing in the second stage in proportions of the biofilm that remained on the liquid surface being 50% or more were almost the same as each other. Accordingly, it was found that it was most efficient when 50% or more of the biofilm was harvested in terms of increasing the total quantity of dry alga bodies of microalgae harvested through culturing in the first stage and the second stage.

The quantity of dry alga bodies of microalgae that remained on the liquid surface after the harvest in the culturing in the first stage was calculated from the above-described quantity of dry alga bodies and the collected area of microalgae as the collected biofilm, and the proliferation ratio of microalgae in the culturing in the second stage was obtained from the quantity of dry alga bodies of the microalgae obtained 14 days after the culturing in the second stage. The results of the proliferation ratios were shown by a polygonal line graph in Fig. 15.

From the above, it was found that it was possible to effectively utilize microalgae since the proliferation ratio of microalgae in the culturing in the second stage was increased as the proportion (survival rate) of the biofilm that remained on the liquid surface after the culturing in the first stage was lower.

In consideration of both the results of the total quantity of dry alga bodies of the harvested microalgae and the proliferation ratio of microalgae, it was found that it was most preferable that the proportion of microalgae that remained on the liquid surface was 50%.

In Fig. 15, the result denoted as "day 0 of culturing is the quantity of dry alga bodies of microalgae obtained through the harvest after the culturing in the first stage as described above. The reason why a finite value could be obtained as the collected amount of microalgae after the culturing in the second stage even in the case where 0% of the biofilm remained on the liquid surface, that is, the total amount of the biofilm was collected is considered to be that only microalgae on the liquid surface of the culture container were collected after the culturing in the first stage, and therefore, microalgae remained on the bottom surface and the side surface of the culture container as they were, and microalgae proliferated therefrom.

### [Example 5]

Pre-culturing and collection of microalgae were performed through the same method as that in Example 1. The period of the pre-culturing in Example 5 was set to 14 days.

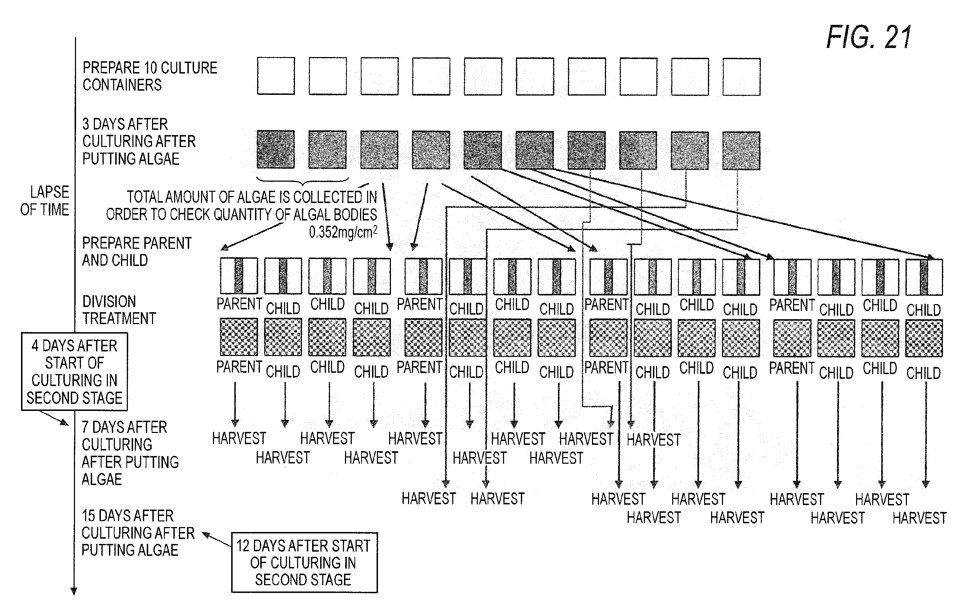

Fig. 21 shows a relationship between "parents" and "children" in culturing from the start of the culturing to harvest.

Culturing in a first stage was performed through the same method as that in Example 1. However, a CSiFF04 medium was used as a medium and the initial use alga body concentration was changed to 50 x 10⁴ cells/mL to prepare 410 mL of a suspended solution of microalgae. In addition, a polystyrene case no. 28, of which the side surface was subjected to a light shielding treatment with black vinyl tape, was used as a culture container. Moreover, 10 containers, into each of which 40 mL of the suspended solution of microalgae, that is, the water depth of the containers becoming 1.5 cm was put, were prepared as shown in Fig. 21 and the amount of light was changed to 15000 lux.

The quantity of dry alga bodies of microalgae as a biofilm formed on a liquid surface was calculated using two culture containers among the 10 culture containers 3 days after the culturing in the first stage. The same method as that in the culturing in the first stage in Example 1 was used as a collecting (harvesting) method. The obtained quantity of dry alga bodies was 0.352 mg/cm².

Collecting of microalgae as a biofilm was performed on 4 culture containers among the remaining 8 culture containers through the same method as that in the culturing in the first stage in Example 1. A nylon film with a width of 1.2 cm (that is, a length of a quarter of the length of a long side of the culture container, that is, the biofilm being divided into 4) was made as a substrate, and the biofilm on a liquid surface was collected as shown in Fig. 5. The collecting was performed three times for one culture container, and the cultured biofilms were applied to different 3 culture containers which were prepared as follows. In addition, in the culturing performed each time, the amount of the collected substance was set to be almost the same as each other as possible.

The collected substance was gently applied to a liquid surface of the polystyrene case no. 28 into which 40 mL of a CSiFF04 medium was previously put, thereby making microalgae as a biofilm collected after the culturing in the first stage float on the liquid surface. The above-described biofilm was subjected to a division treatment in a manner that the entire culture container was shaken, to divide the biofilm into a suitable size. Three culture containers were used with respect to the one culture container. That is, the total number of culture containers was 16. Culturing in a second stage to be described later was performed on the remaining 4 culture containers among the 8 culture containers in a state where partial collection was not performed thereon, and collection was performed 4 days and 12 days after the culturing in the second stage. The result was denoted as "no partial harvest" in Fig. 16.

With respect to the remaining 4 culture containers other than the above-described culture containers which were denoted as "no partial harvest", a part of the biofilm which was subjected to a division treatment through the following procedure was transferred to new 3 culture containers (which were denoted as "child 1", "child 2", and "child 3" in Fig. 16), and culturing in a second stage to be described later was performed. At the same time, the culturing in the second stage to be described later was also performed on the four culture containers (which were denoted as "parent" in Fig. 16), each of which includes a biofilm that remained after the part of the biofilm subjected to the division treatment was collected.

Specifically, the biofilm which was subjected to the division treatment and existed on the liquid surface of the culture container was collected again using the nylon film which was used in the collection after the culturing in the first stage, and the biofilm was transferred from the culture container of "parent" to the culture container of "child". The biofilm referred to herein was a biofilm obtained after collection from the liquid surface had been performed once, and therefore, the film-like structure on the liquid surface slightly collapsed. Thus it was difficult to control the collected amount compared to previous collection.

The collected substance was gently applied to a liquid surface of the polystyrene case no. 28 ("culture container of "child"), into which 40 mL of a CSiFF04 medium was previously put, thereby making the collected substance float on the liquid surface. The biofilm floating on the liquid surface was subjected to a division treatment in a manner that the entire culture container of "child" was shaken. After the division treatment, the biofilm became a large number of biofilms having smaller sizes by setting the sizes thereof from about several mm² to 1 cm² as maximum values.

The same operation as that described above was performed to prepare new 3 culture containers ("child 1" to "child 3") in which a part of a biofilm subjected to a division treatment in one culture container was made to float on the liquid surface, from the biofilm.

In addition, the same operation was performed on the remaining 3 culture containers to obtain 16 culture containers in total.

With respect to 20 culture containers in total which were adjusted as described above, each of 2 vacuum desiccators, into which 8 culture containers were put, and one vacuum desiccator, into which 4 culture containers were put, were prepared, and culturing in a second stage was started under the same culture conditions as those in the culturing in the first stage.

Collection (harvest) of the biofilm formed on the liquid surface was performed through the same method as that in the culturing in the second stage in Example 1, 4 days and 12 days after the start of the culturing in the second stage. With respect to each of the culture containers which was denoted as "no partial harvest", "parent", and "child 1" to "child 3", the results of the quantities of dry alga bodies of biofilms collected 4 days and 12 days after the start of the culturing in the second stage were shown in Fig. 16.

As described above, in the drawing, the "no partial harvest" indicates the case where harvest was not performed in the middle of culturing, the "parent" indicates the case where culturing was continued using a biofilm remaining on a liquid surface after partial collection, and the "child" indicates the case where culturing was performed by performing a division treatment after making a collected substance after the partial collection float on a liquid surface.

A biofilm was formed on the liquid surface of the culture container of a child even 4 days after the start of the culturing in the second stage, and formation of a biofilm as a three-dimensional structure could be seen 12 days after the start of the culturing in the second stage. When comparing the "parent" and the "child 1" to the "child 3" with the "no partial harvest", the quantity of dry alga bodies of each of the "parent" and the "child 1" to the "child 3" was small 12 days after the start of the culturing in the second stage. However, the quantity of dry alga bodies of each of the "parent" and the "child 1" to the "child 3" was combined, and the harvested amount thereof was 30% greater than that of the quantity of dry alga bodies of the "no partial harvest".

These results show that through partial harvest of a biofilm formed on a liquid surface, it is possible to perform culturing by transferring the biofilm to a plurality of culture containers and to perform culturing so as to form a biofilm on a liquid surface in each of the culture containers, and the culture efficiency of microalgae in the total harvested amount is better than in the case of continuous culturing in one culture container without performing partial harvest.

### Industrial Applicability

According to the method of culturing microalgae according to the present invention, it is possible to perform subsequent culturing without preparing a suspended solution of microalgae. Furthermore, it is unnecessary to introduce the suspended solution of microalgae to the next culture container and to stir the entire medium existing in the culture container. Moreover, it is possible to substantially stir or disperse only a biofilm of microalgae, which is on a liquid surface, on the liquid surface to thereby efficiently perform culturing. Furthermore, it is possible to form a biofilm on the liquid surface, and thus, it is extremely easy to collect microalgae compared to floating culture in the related art. That is, in the present invention, a biofilm formed of an aggregate of microalgae floats on a liquid surface in a stage of collecting microalgae and the biofilm is set as a subject to be collected. Therefore, it is unnecessary to collect microalgae from a large amount of medium unlike in the related art, and only the biofilm on the liquid surface and water contained in the biofilm are set as a subject to be collected. For this reason, it is possible to greatly reduce the collection cost of microalgae. In addition, it is unnecessary to handle a large amount of liquid medium and to use a large amount of water.

The present invention has been described in detail or with reference to a specific embodiment. It is obvious to those skilled in the art that it is possible to make various modifications or corrections without departing from the gist or the scope of the present invention.

The present application is based on Japanese Patent Application (No. 2012-268781), filed December 7,2012, the entire disclosure of which is incorporated herein by reference. Reference Signs List

1 second culture container
2 first culture container

## Claims

1. A method of culturing microalgae, comprising:
culturing in a first stage in which microalgae obtained through a purification process are cultured in a culture solution within a first culture container to form a biofilm as a film-like structure or a three-dimensional structure which is formed of the microalgae on a liquid surface of the culture solution; and
culturing in a second stage in which microalgae are cultured on a liquid surface using at least a part of the biofilm as a film-like structure or a three-dimensional structure which is formed on the liquid surface of the culture solution, as seed algae.

2. The method of culturing microalgae according to claim 1,
wherein the culturing in the second stage is performed by collecting a part of or the entirety of the biofilm as a film-like structure or a three-dimensional structure which is formed on the liquid surface in the culturing in the first stage, from the first culture container, transferring the collected biofilm into a second culture container, and using the biofilm as seed algae in a culture solution in the second culture container.

3. The method of culturing microalgae according to claim 1 or 2,
wherein the culturing in the second stage is performed by collecting a part of the biofilm as a film-like structure or a three-dimensional structure which is formed on the liquid surface in the culturing in the first stage, plural times using a substrate, transferring the collected biofilms into a plurality of second culture containers, and using the biofilms as seed algae in culture solutions in the respective plurality of second culture containers.

4. The method of culturing microalgae according to claim 1,
wherein the culturing in the second stage is performed by collecting a part of the biofilm as a film-like structure or a three-dimensional structure which is formed on the liquid surface in the culturing in the first stage, from the first culture container, and using a biofilm remaining in the first culture container, as seed algae, in the culture solution in the first culture container.

5. The method of culturing microalgae according to any one of claims 1 to 4, further comprising:
performing a division treatment, in which the biofilm as a film-like structure or a three-dimensional structure which is formed on the liquid surface in the culturing in the first stage is divided, between the culturing in the first stage and the culturing in the second stage.

6. The method of culturing microalgae according to claim 5,
wherein the division treatment is performed on a liquid surface where a region, in which a biofilm substantially exists, and a region, in which a biofilm does not substantially exist, coexist.

7. The method of culturing microalgae according to claim 6,
wherein the divided biofilm is moved to the region in which a biofilm does not substantially exist, through the division treatment.

8. The method of culturing microalgae according to any one of claims 1 to 7,
wherein the biofilm as a film-like structure or a three-dimensional structure which is formed on the liquid surface in the culturing in the first stage is collected using one sheet of a substrate which has a plurality of sites for collecting a biofilm.

9. The method of culturing microalgae according to any one of claims 2, 3, and 5 to 8,
wherein the area of the second culture container is larger than that of the first culture container.

10. The method of culturing microalgae according to claim 9,
wherein the first culture container, which has the biofilm as a film-like structure or a three-dimensional structure which is formed on the liquid surface after the culturing in the first stage, is allowed to sink in the second culture container to float the biofilm on the liquid surface of the second culture container and to transfer the biofilm to the second culture container.

11. The method of culturing microalgae according to any one of claims 1 to 10,
wherein when collecting a part of the biofilm as a film-like structure or a three-dimensional structure which is formed on the liquid surface in the culturing in the first stage from the first culture container, the biofilm corresponding to 25% to 75% of the area of the liquid surface is collected.

12. The method of culturing microalgae according to any one of claims 1 to 11,
wherein both the culturing in the first stage and the culturing in the second stage are stationary culture.

13. The method of culturing microalgae according to any one of claims 1 to 12,
wherein the biofilm as a film-like structure or a three-dimensional structure which is formed on the liquid surface in the culturing in the first stage is collected by being transferred to or deposited on the surface of a substrate.

14. The method of culturing microalgae according to any one of claims 1 to 13,
wherein the microalgae are green algae.

15. The method of culturing microalgae according to any one of claims 1 to 14,
wherein the microalgae are microorganisms containing oil.

16. The method of culturing microalgae according to any one of claims 1 to 15,
wherein the microalgae belongs to the genus Botryococcus (Botryococcus sp.).

17. The method of culturing microalgae according to claim 16,
wherein the microalgae belongs to Botryococcus sudeticus.

18. The method of culturing microalgae according to claim 17,
wherein the microalgae are Botryococcus sudeticus AVFF007 strains (deposition number of FERM BP-11420).

19. A biofilm which is formed on a liquid surface through the method of culturing microalgae according to any one of claims 1 to 18.

20. Biomass which is obtained from the biofilm according to claim 19.

21. Oil which is obtained from the biofilm according to claim 19.

22. A method of producing biomass derived from microalgae, comprising:
the method of culturing microalgae according to any one of claims 1 to 18.


SEQUENCE LISTING

<110>  FUJIFILM Corporation

<120>  Method for culturing microalgae, biofilm formed on liquid level
       by the culturing method, biomass and oil content obtained from
       the biofilm and production method of biomass derived from
       microalgae

<130>  W105239
<150>  JP 2012-268781
<151>  2012-12-07
<160>  1

<170>  PatentIn version 3.5

<210>  1
<211>  1111
<212>  DNA
<213>  Botryococcus sudeticus

<400>  1
cggtcgcttc ggtgagtact gctatggcct tcctttctac gggggacggg ctcctgggat      60

taacttctcg ggatccggat tccgtgatga tactttgagt aaattggagt gttcaaagca     120

agcctgcgct ctgaacattt tagcatggaa tatcacgata ggactctggc ctatcttgtt     180

ggtctgtagg accagagtaa tgattaagag ggacagtcgg gggcattcgt atttcattgt     240

cagaggtgaa attcttggat ttatgaaaga cgaactactg cgaaagcatt tgccaaggat     300

gttttcatta atcaagaacg aaagttgggg gctcgaagac gattagatac cgtcgtagtc     360

tcaaccataa acgatgccga ctagggatta gcagacgttt cattgatgac tctgctagca     420

ccttatgtga aaacaaagtt tttgggttcc ggggggagta tggtcgcaag gctgaaactt     480

aaaggaattg acggaagggc accaccaggc gtggagcctg cggcttaatt tgactcaaca     540

cgggaaaact taccaggtcc agacatagtg aggattgaca gattgagagc tctttcttga     600

ttctatgggt ggtggtgcat ggccgttctt agttggtggg ttgccttgtc aggttgattc     660

cggtaacgaa cgagacctca gctcctaaat agtccatgcc gccttttggc ggtatgctga     720

cttcttagag ggactattgg cgttctagcc aatggaagta tgaggcaata acaggtctgt     780

gatgccctta gatgttctgg gccgcacgcg cgctacactg atgcgttcaa cgagcctatc     840

cttgaccgag aggtccgggt aatctttgaa accgcatcgt gatggggcta gactattgca     900

attattagtc tttaacgagg aatgcctagt aagcgcgatt catcagatcg cgttgattac     960

gtccctggta cacaccgccc gtcgctccta ccgattgggt gtgctggtga agtgttcgga    1020

tcggcaaagc tgggggcaac tcttgcattg ccgagaagtt cattaaaccc tcccacctag    1080

aggaaggaga agtcgtaaca aggtttccgt a                                   1111